# EUROPEAN PATENT APPLICATION

(11) **EP 4 438 722 A1**
(43) Date of publication of application: **02.10.2024**
(21) Application number: 22898584.2
(22) Date of filing: 22.11.2022
(51) Int. Cl.: C12N 5/0783

(54) **HUMAN INDUCIBILITY CONTROLLABLE T-CELL AND METHOD FOR PREPARING SAME**

(30) Priority: 24.11.2021 JP 2021190127
(71) Applicant: Regcell Co., Ltd., Kyoto-shi, Kyoto 603-8436 (JP)
(72) Inventor: MIKAMI, Norihisa, Kyoto-shi, Kyoto 603-8436 (JP); SAKAGUCHI, Shimon, Kyoto-shi, Kyoto 603-8436 (JP)
(74) Representative: Vossius & Partner Patentanwälte Rechtsanwälte mbB
(86) International application number: PCT/JP2022/043220
(87) International publication number: WO 2023/095801

(57) **Abstract**

According to the present disclosure, there is provided an inducible regulatory T cell having high functionality and a stable immunosuppressive function. The present disclosure provides an inducible regulatory human T cell having at least one feature selected from the group consisting of FoxP3 positivity, CTLA4 positivity, NT5E positivity, ITGAE (CD103) positivity, AREG positivity, CD172g positivity, and CD26 positivity.

## Description

### Technical Field

The present disclosure relates to a human inducible regulatory T cell having high functionality and a method for preparing the same.

### Background Art

As an important feature of a CD25-positive CD4-positive regulatory T cell intrinsic to the immune system, a transcription factor FoxP3 is specifically expressed, and the occurrence and differentiation of the regulatory T cell and the suppression function may be impaired by the defect or mutation of FoxP3. The regulatory T cell suppresses the immune system by expression of various genes such as CTLA4 and IL-10 in addition to FoxP3. It is considered that epigenetic states such as DNA demethylation contribute to comprehensive gene expression control of the regulatory T cell including stable expression of FoxP3, and they correlate with the phenotype of the functional regulatory T cell.

### Summary of Invention

### Solution to Problem

The present inventors have found for the first time that when a regulatory T cell is induced from a human peripheral T cell, a stable T cell is induced from the human peripheral T cell by stimulation using an anti-CD3 antibody, then dormant culturing is performed, anti-CD3 antibody stimulation is performed again and culturing is performed, and dormant culturing is further performed, whereby a regulatory T cell having high expression of suppressive molecules and a high suppression function can be induced.

Therefore, the present disclosure provides the following.

### (Item X1)

An inducible regulatory human T cell having at least one feature selected from the group consisting of FoxP3 positivity, CTLA4 positivity, NT5E positivity, ITGAE (CD103) positivity, AREG positivity, CD 172g positivity, and CD26 positivity.

### (Item X1A)

An inducible regulatory human T cell which is NT5E-positive.

### (Item X1B)

An inducible regulatory human T cell which is ITGAE (CD103)-positive.

### (Item X1C)

An inducible regulatory human T cell which is AREG-positive.

### (Item X1D)

An inducible regulatory human T cell which is CD172g-positive.

### (Item X1E)

An inducible regulatory human T cell which is CD26-positive.

### (Item X1F)

An inducible regulatory human T cell which is CTLA4-positive.

### (Item X1G1)

An inducible regulatory human T cell having at least two features selected from the group consisting of CTLA4 positivity, NT5E positivity, ITGAE (CD103) positivity, AREG positivity, CD 172g positivity, and CD26 positivity.

### (Item X1G2)

An inducible regulatory human T cell having at least three features selected from the group consisting of CTLA4 positivity, NT5E positivity, ITGAE (CD103) positivity, AREG positivity, CD 172g positivity, and CD26 positivity.

### (Item X1G3)

An inducible regulatory human T cell having at least four features selected from the group consisting of CTLA4 positivity, NT5E positivity, ITGAE (CD103) positivity, AREG positivity, CD172g positivity, and CD26 positivity.

### (Item X1G4)

An inducible regulatory human T cell having at least five features selected from the group consisting of CTLA4 positivity, NT5E positivity, ITGAE (CD103) positivity, AREG positivity, CD172g positivity, and CD26 positivity.

### (Item X1G5)

An inducible regulatory human T cell having all features of CTLA4 positivity, NT5E positivity, ITGAE (CD103) positivity, AREG positivity, CD172g positivity, and CD26 positivity.

### (Item X2)

The inducible regulatory human T cell according to any one of the preceding items and claim 1, which is at least CTLA4-positive and FoxP3-positive.

### (Item X3)

The inducible regulatory human T cell according to any one of the preceding items, which is at least CD172g-positive and/or CD26-positive.

### (Item X4)

The inducible regulatory human T cell according to any one of the preceding items, wherein the CNS2 site of the FOXP3 gene is demethylated.

### (Item X5)

The inducible regulatory human T cell according to any one of the preceding items, which is CD4-positive or CD8-positive.

### (Item X6)

The inducible regulatory human T cell according to any one of the preceding items, which is obtained from or derived from a human peripheral blood T cell or a human tissue-derived T cell.

### (Item X7)

An inducible regulatory human T cell obtained by a method including the steps of:
(a) stimulating a CD4-positive T cell or a CD8-positive T cell in human peripheral blood in a first basal medium for about 1 to about 5 days;
(b) dormant culturing the cell obtained in step (a) in a medium containing IL-2 for at least about 1 to about 3 days;
(c) stimulating the cell obtained in step (b) in a second basal medium for about 1 to about 5 days; and
(d) dormant culturing the cell obtained in step (c) in a medium containing IL-2 for at least about 1 to about 3 days.

### (Item X8)

A cell population comprising the inducible regulatory human T cell according to any one of the preceding items, wherein the cell population has a proportion of at least one feature selected from the group consisting of FoxP3 positivity, CTLA4 positivity, NT5E positivity, ITGAE (CD103) positivity, AREG positivity, CD172g positivity, and CD26 positivity of about 50% or more.

### (Item X8A)

A cell population comprising an inducible regulatory human T cell, wherein the cell population has a proportion of NT5E positivity of about 50% or more.

### (Item X8B)

A cell population comprising an inducible regulatory human T cell, wherein the cell population has a proportion of ITGAE (CD103) positivity of about 50% or more.

### (Item X8C)

A cell population comprising an inducible regulatory human T cell, wherein the cell population has a proportion of AREG positivity of about 50% or more.

### (Item X8D)

A cell population comprising an inducible regulatory human T cell, wherein the cell population has a proportion of CD172g positivity of about 50% or more.

### (Item X8E)

A cell population comprising an inducible regulatory human T cell, wherein the cell population has a proportion of CD26 positivity of about 50% or more.

### (Item X8F)

A cell population comprising an inducible regulatory human T cell, wherein the cell population has a proportion of CTLA4 positivity of about 50% or more.

### (Item X8G1)

A cell population comprising an inducible regulatory human T cell, wherein the cell population has each proportion of at least two features selected from the group consisting of CTLA4 positivity, NT5E positivity, ITGAE (CD103) positivity, AREG positivity, CD172g positivity, and CD26 positivity of about 50% or more.

### (Item X8G2)

A cell population comprising an inducible regulatory human T cell, wherein the cell population has each proportion of at least three features selected from the group consisting of CTLA4 positivity, NT5E positivity, ITGAE (CD103) positivity, AREG positivity, CD172g positivity, and CD26 positivity of about 50% or more.

### (Item X8G3)

A cell population comprising an inducible regulatory human T cell, wherein the cell population has each proportion of at least four features selected from the group consisting of CTLA4 positivity, NT5E positivity, ITGAE (CD103) positivity, AREG positivity, CD172g positivity, and CD26 positivity of about 50% or more.

### (Item X8G4)

A cell population comprising an inducible regulatory human T cell, wherein the cell population has each proportion of at least five features selected from the group consisting of CTLA4 positivity, NT5E positivity, ITGAE (CD103) positivity, AREG positivity, CD172g positivity, and CD26 positivity of about 50% or more.

### (Item X8G5)

A cell population comprising an inducible regulatory human T cell, wherein the cell population has each proportion of all features of CTLA4 positivity, NT5E positivity, ITGAE (CD103) positivity, AREG positivity, CD172g positivity, and CD26 positivity of about 50% or more.

### (Item X9)

The cell population according to any one of the preceding items, wherein the proportions of at least CTLA4 positivity and FoxP3 positivity each are about 50% or more.

### (Item X10)

The cell population according to any one of the preceding items, wherein the proportions of at least CD172g positivity and/or CD26 positivity each are about 50% or more.

### (Item X11)

The cell population according to any one of the preceding items, wherein the proportion of at least one feature in the cell population is about 60% or more.

### (Item X12)

The cell population according to any one of the preceding items, wherein the proportion of at least one feature in the cell population is about 80% or more.

### (Item X12a)

The cell population according to any one of the preceding items, wherein the proportion of FoxP3 strong positivity is about 50% or more.

### (Item X13)

The cell population according to any one of the preceding items, wherein about 90% or more of the cell population is T cells.

### (Item X14)

A pharmaceutical composition comprising the inducible regulatory human T cell according to any one of the preceding items or the cell population according to any one of the preceding items.

### (Item X15)

A regenerative medical material or product comprising the inducible regulatory human T cell according to any one of the preceding items or the cell population according to any one of the preceding items.

### (Item X16)

A method for preparing an inducible regulatory human T cell, the method comprising the steps of:
(a) stimulating a CD4-positive T cell or a CD8-positive T cell in human peripheral blood in a first basal medium for about 1 to about 5 days;
(b) dormant culturing the cell obtained in step (a) in a medium containing IL-2 for at least about 1 to about 3 days;
(c) stimulating the cell obtained in step (b) in a second basal medium for about 1 to about 5 days; and
(d) dormant culturing the cell obtained in step (c) in a medium containing IL-2 for at least about 1 to about 3 days.

### (Item X17)

The method according to any one of the preceding items, wherein the first basal medium comprises at least one factor selected from the group consisting of an anti-CD3 antibody, TGF-β1, IL-2, retinoic acid, a CDK8 inhibitor, a CDK19 inhibitor, a CDK8/19 inhibitor, and ascorbic acid.

### (Item X18)

The method according to any one of the preceding items, wherein the first basal medium comprises an anti-CD3 antibody, TGF-β1, IL-2, retinoic acid, a CDK8 inhibitor, a CDK19 inhibitor, a CDK8/19 inhibitor, and ascorbic acid.

### (Item X19)

The method according to any one of the preceding items, wherein the second basal medium comprises at least one factor selected from the group consisting of an anti-CD3 antibody, TGF-β1, IL-2, retinoic acid, a CDK8 inhibitor, a CDK19 inhibitor, and a CDK8/19 inhibitor.

### (Item X20)

The method according to any one of the preceding items, wherein the second basal medium comprises an anti-CD3 antibody, TGF-β1, IL-2, retinoic acid, a CDK8 inhibitor, a CDK19 inhibitor, and a CDK8/19 inhibitor.

### (Item X21)

The method according to any one of the preceding items, wherein step (a) is to stimulate the CD4-positive T cell or the CD8-positive T cell in the first basal medium for about 3 days.

### (Item X22)

The method according to any one of the preceding items, wherein step (b) is to dormant culture the cell obtained in step (a) in the medium containing IL-2 for at least about 2 days.

### (Item X23)

The method according to any one of claims 16 to 22, wherein step (c) is to stimulate the cell obtained in step (b) in the second basal medium for about 3 days.

### (Item X24)

The method according to any one of the preceding items, wherein step (d) is to dormant culture the cell obtained in step (c) in the medium containing IL-2 for at least about 2 days.

### (Item X25)

An inducible regulatory human T cell generated by the method according to any one of the preceding items or a cell population comprising the inducible regulatory human T cell.

The present disclosure also provides the following.

### (Item 1)

An inducible regulatory T cell having at least one feature selected from the group consisting of CTLA4 positivity, NT5E positivity, ITGAE (CD103) positivity, and AREG positivity.

### (Item 2)

The inducible regulatory T cell according to any one of the preceding items, which has at least two features selected from the group consisting of CTLA4 positivity, NT5E positivity, ITGAE (CD103) positivity, and AREG positivity.

### (Item 3)

The inducible regulatory T cell according to any one of the preceding items, which is at least CTLA4-positive.

### (Item 4)

The inducible regulatory T cell according to any one of the preceding items, wherein the CNS2 site of the FOXP3 gene is demethylated.

### (Item 5)

The inducible regulatory T cell according to any one of the preceding items, which is CD4-positive or CD8-positive.

### (Item 6)

The inducible regulatory T cell according to any one of the preceding items, which is obtained from or derived from a human peripheral blood T cell or a human tissue-derived T cell.

### (Item 7)

An inducible regulatory T cell obtained by a method including the steps of:
(a) stimulating a CD4-positive T cell or a CD8-positive T cell in peripheral blood in a first basal medium for about 1 to about 5 days;
(b) dormant culturing the cell obtained in step (a) in a medium containing IL-2 for at least about 1 to about 3 days;
(c) stimulating the cell obtained in step (b) in a second basal medium for about 1 to about 5 days; and
(d) dormant culturing the cell obtained in step (c) in a medium containing IL-2 for at least about 1 to about 3 days.

### (Item 8)

A cell population comprising T cells, wherein about 50% or more of the T cells of the cell population is the inducible regulatory T cell according to any one of the preceding items.

### (Item 9)

The cell population according to claim 8, wherein about 80% or more of the T cells of the cell population is the inducible regulatory T cell according to any one of the preceding items.

### (Item 10)

The cell population according to any one of the preceding items, wherein the T cell in the cell population is a regulatory T cell.

### (Item 11)

The cell population according to any one of the preceding items, wherein about 90% or more of the cell population is T cells.

### (Item 12)

A pharmaceutical composition comprising the inducible regulatory T cell according to any one of the preceding items or the cell population according to any one of the preceding items.

### (Item 13)

A regenerative medical material or product comprising the inducible regulatory T cell according to any one of the preceding items or the cell population according to any one of the preceding items.

### (Item A1)

A method for preparing an inducible regulatory T cell, the method comprising the steps of:
(a) stimulating a CD4-positive T cell or a CD8-positive T cell in peripheral blood in a first basal medium for about 1 to about 5 days;
(b) dormant culturing the cell obtained in step (a) in a medium containing IL-2 for at least about 1 to about 3 days;
(c) stimulating the cell obtained in step (b) in a second basal medium for about 1 to about 5 days; and
(d) dormant culturing the cell obtained in step (c) in a medium containing IL-2 for at least about 1 to about 3 days.

### (Item A2)

The method according to any one of the preceding items, wherein the first basal medium comprises at least one factor selected from the group consisting of an anti-CD3 antibody, TGF-β1, IL-2, retinoic acid, a CDK8 inhibitor, a CDK19 inhibitor, a CDK8/19 inhibitor, and ascorbic acid.

### (Item A3)

The method according to any one of the preceding items, wherein the first basal medium comprises an anti-CD3 antibody, TGF-β1, IL-2, retinoic acid, a CDK8 inhibitor, a CDK19 inhibitor, a CDK8/19 inhibitor, and ascorbic acid.

### (Item A4)

The method according to any one of the preceding items, wherein the second basal medium comprises at least one factor selected from the group consisting of an anti-CD3 antibody, TGF-β1, IL-2, retinoic acid, a CDK8 inhibitor, a CDK19 inhibitor, and a CDK8/19 inhibitor.

### (Item A5)

The method according to any one of the preceding items, wherein the second basal medium comprises an anti-CD3 antibody, TGF-β1, IL-2, retinoic acid, a CDK8 inhibitor, a CDK19 inhibitor, and a CDK8/19 inhibitor.

### (Item A6)

The method according to any one of the preceding items, wherein step (a) is to stimulate the CD4-positive T cell or the CD8-positive T cell in the first basal medium for about 3 days.

### (Item A7)

The method according to any one of the preceding items, wherein step (b) is to dormant culture the cell obtained in step (a) in the medium containing IL-2 for at least about 2 days.

### (Item A8)

The method according to any one of the preceding items, wherein step (c) is to stimulate the cell obtained in step (b) in the second basal medium for about 3 days.

### (Item A9)

The method according to any one of the preceding items, wherein step (d) is to dormant culture the cell obtained in step (c) in the medium containing IL-2 for at least about 2 days.

### (Item A10)

An inducible regulatory T cell generated by the method according to any one of the preceding items or a cell population comprising the inducible regulatory T cell.

The present disclosure is intended so that one or more of the aforementioned characteristics can be provided not only as the explicitly disclosed combinations, but also as other combinations thereof. Additional embodiments and advantages of the present disclosure are recognized by those skilled in the art by reading and understanding the following detailed description as needed.

The characteristics and significant action and effect of the present disclosure other than those described above will be clear to those skilled in the art by referring to the following Embodiments of the Invention section and the drawings.

### Advantageous Effects of Invention

The present disclosure can provide a method of inducing a regulatory T cell having high functionality from a human peripheral T cell in vitro. The regulatory T cell induced by the method of the present disclosure highly expresses a gene associated with the regulatory T cell and has a strong suppressive ability. Therefore, the T cell can be used as the regulatory T cell for treatment and prevention of various immune diseases and inflammatory diseases such as autoimmune diseases.

A functional regulatory T cell can be induced in vitro from a human peripheral T cell by the method of the present disclosure. The regulatory T cell obtained by the method of the present disclosure has functionality (for example, has a high suppression function) and is stable as the regulatory T cell. That is, by the method of the present disclosure, it is possible to stably express FoxP3, which is a master gene of a regulatory T cell, from a human peripheral T cell and to induce a regulatory T cell having high functionality.

### Brief Description of Drawings

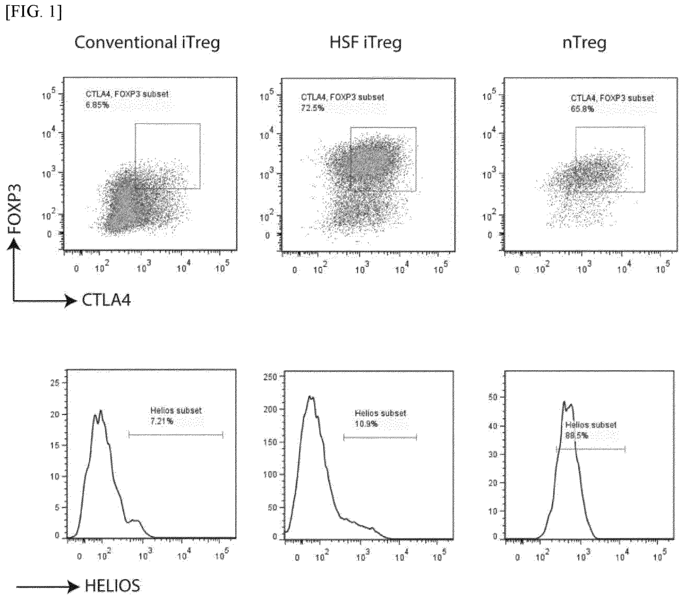

FIG. 1 is a view showing phenotypic analysis (flow cytometry) of one embodiment of an inducible regulatory T cell (also referred to as a human high-functional stable iTreg (HSF iTreg) cell) of the present disclosure. The inducible regulatory T cell of the present disclosure (HSF-iTreg in the figure) was prepared from a human CD4-positive T cell, and the expression of FOXP3, CTLA4, and Helios was analyzed by a flow cytometry method. A cell stimulated with normal nTreg (CD4-positive CD25-positive T cell) and a normal iTreg (Conventional iTreg: cell in which CD4-positive T cell was stimulated with CD3/CD28 in the presence of IL-2 and TGF-β1 for 3 days) were compared.

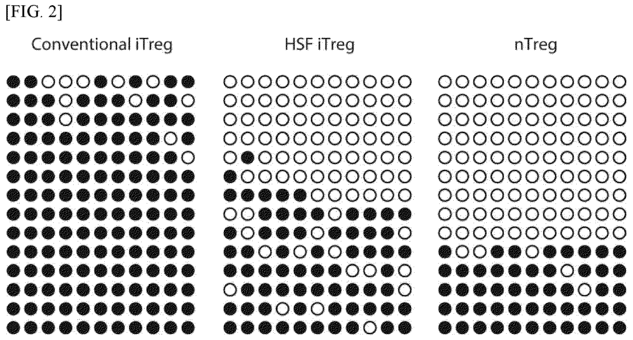

FIG. 2 is a view showing epigenome analysis (bisulfite method) of one embodiment of the iTreg (HSF iTreg) cell of the present disclosure. For each cell in FIG. 1, the demethylation state of the FOXP3 CNS2 region was analyzed by a bisulfite method.

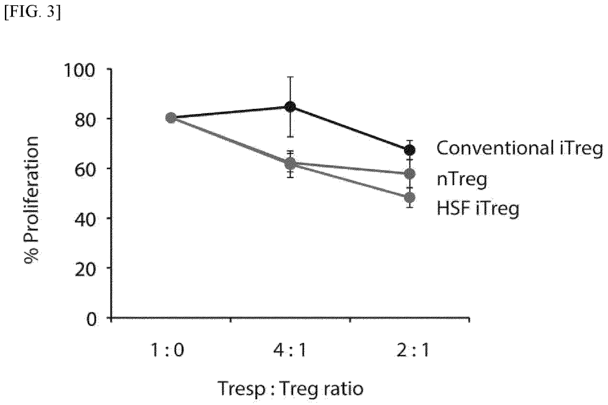

FIG. 3 is a view showing suppressive ability analysis (in vitro suppression assay) of one embodiment of the iTreg (HSF iTreg) cell of the present disclosure. The in vitro suppressive ability of each cell in FIG. 1 was analyzed. A human CD4-positive T cell was stained with a Cell Trace Violet reagent, and mixed and cultured for 3 days at a constant ratio with each cell in FIG. 1 in the presence of a Treg Suppression Inspector (Miltenyi Biotec) (5 µL/well), and the Cell Trace Violet intensity was analyzed by a flow cytometry method.

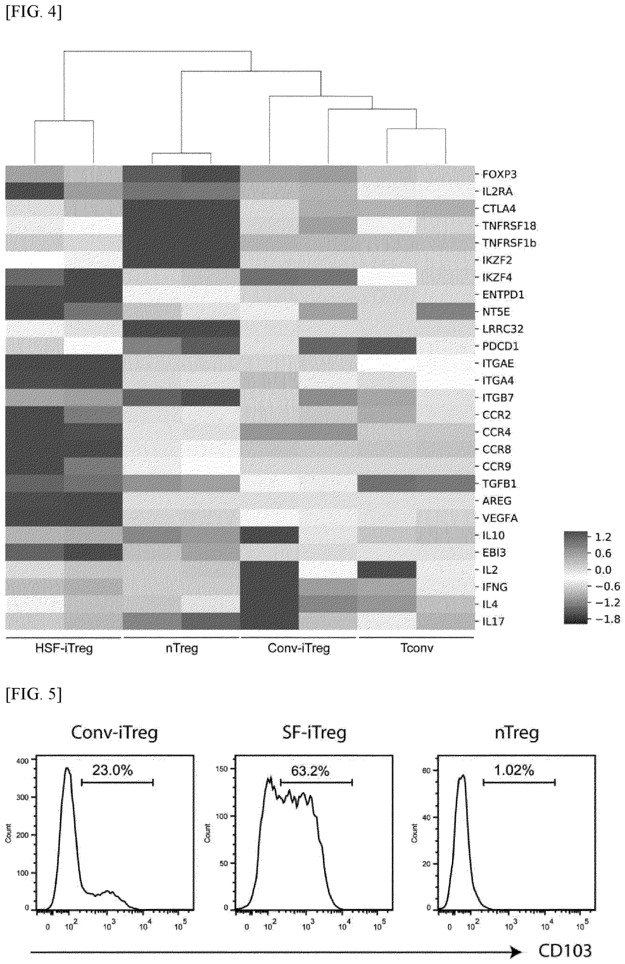

FIG. 4 is a view showing gene expression pattern analysis (RAN-seq method) of one embodiment of the iTreg (HSF iTreg) cell of the present disclosure. For each cell in FIG. 1 and a FOXP3-negative cell (Tconv) in which the CD4-positive T cell was stimulated in the presence of IL-2, a comprehensive gene expression pattern was analyzed by an RNA sequencing method.
FIG. 5 is a view showing CD103 expression (flow cytometry method) of one embodiment of the iTreg (HSF iTreg) cell of the present disclosure. For each cell in FIG. 1, CD103 expression in the FOXP3-positive cell was analyzed by a flow cytometry method.

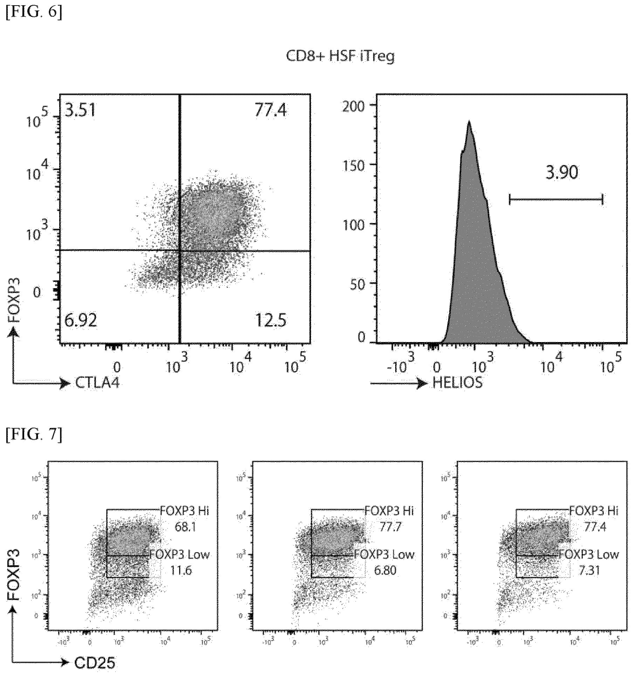

FIG. 6 is a view showing phenotypic analysis (flow cytometry method) of one embodiment of the iTreg (HSF iTreg) cell of the present disclosure prepared from a human CD8-positive T cell. The regulatory T cell of the present disclosure was prepared from a human CD8-positive T cell, and the expression of FOXP3, CTLA4, and Helios was analyzed by a flow cytometry method.
FIG. 7 is a view showing CD25 expression and FOXP3 expression (flow cytometry method) of one embodiment of the iTreg (HSF iTreg) cell of the present disclosure. The expression intensity of CD25 and FOXP3 was analyzed by a flow cytometry method using a BD FACSLyric flow cytometer.

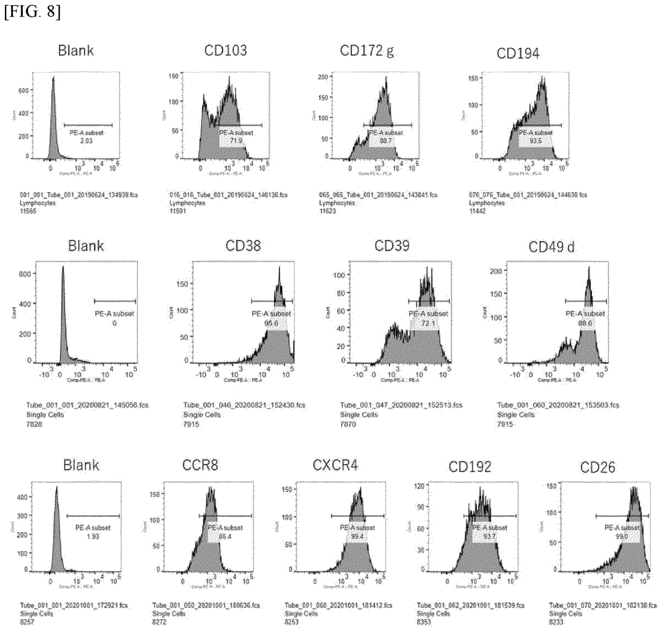

FIG. 8 is a view showing phenotypic analysis of one embodiment of the iTreg (HSF iTreg) cell of the present disclosure. The inducible regulatory T cell (HSF-iTreg) of the present disclosure was prepared, and expression of CD172g, CD26, and the like was analyzed.

### Description of Embodiments

Hereinafter, the present disclosure will be described while showing the best mode. Throughout the entire present specification, a singular expression should be understood as encompassing the concept thereof in the plural form, unless specifically noted otherwise. Thus, singular articles (for example, "a", "an", "the", and the like in the case of English) should also be understood as encompassing the concept thereof in the plural form, unless specifically noted otherwise. The terms used in the present specification should be understood as being used in the meaning that is commonly used in the art, unless specifically noted otherwise. Therefore, unless defined otherwise, all terminologies and scientific technical terms that are used in the present specification have the same meaning as the general understanding of those skilled in the art to which the present disclosure pertains. In the case of a contradiction, the present specification (including the definitions) takes precedence

Hereinafter, definitions and/or basic technical contents of terms particularly used in the present specification will be described as appropriate.

In the present specification, "about" refers to a range of ±10% from the numerical value that is described subsequent to "about". For example, "about 20" includes "18 to 22". The range of numerical values includes all numerical values between both ends and numerical values of the both ends. When a range is accompanied with the term "about", it is intended that the values of the both ends are accompanied with the term "about". Thus, for example, "about 20 to 30" includes "18 to 33".

In the present specification, when a gene name and a product thereof are expressed, they may refer to both a gene and a protein in the case of being expressed in all capital letters, unlike normal usage. For example, FOXP3 gene and FOXP3 protein may be used separately, and when expressed as FoxP3, both (all) of the concept and the practice of the gene or protein are indicated.

In the present specification, the "regulatory T cell" is a T cell that is positive for the expression of FoxP3. In the present specification, the regulatory T cell may also be referred to as "Treg". Treg includes a naturally occurring regulatory T cell (nTreg), an inducible regulatory T cell (iTreg), and the like. The regulatory T cell usually can have a variety of functions (for example, immunosuppressive function).

In the present specification, the "inducible regulatory T cell (iTreg)" refers to a regulatory T cell that is negative for the expression of IKZF2 (Helios). iTreg is usually obtained by differentiation induction from a naive CD4-positive T cell or the like.

In the present specification, the "naturally occurring regulatory T cell (nTreg)" is a regulatory T cell that is positive for the expression of IKZF2 (Helios) and CTLA4. The naturally occurring regulatory T cell is usually a cell present in a living body.

In the present specification, the "peripheral T cell" refers to a T cell existing outside the thymus, and can be obtained from peripheral blood, lymph nodes, or other tissues. In the present specification, the term "peripheral T cell" only needs to include a peripheral T cell in the cell group, and the T cell does not need to be isolated. In addition to the T cell such as a peripheral blood mononuclear cell (PBMC), cell fractions containing various lymphocytes may be used.

In the present specification, the term "flow cytometry" refers to a technique for measuring the number of cells, individuals, and other biological particles suspended in a liquid and individual physical/chemical/biological attributes. An apparatus using this technique is referred to as a "flow cytometer". In the present disclosure, a cell marker (for example, FoxP3, CTLA4, Helios, CD103, or the like) of "positive" or "negative" is defined by flow cytometry, as commonly used in the art. More specifically, in the flow cytometry, cells are flushed in line and the number of cells is counted by a spectroscopic method. For example, the number of target cells are counted by irradiating the cells labeled by the fluorescence or the luminescent enzyme with laser beams, and thereby detecting the fluorescence or the emission signals from the cells with a detector such as a photodiode. The detection result with the detector can be loaded into a computer to create and display a two-dimensional plot. The presence or absence of target cells and the number of target cells can be thereby easily grasped.

In the present specification, "demethylation" refers to removal of methylation modification of adenine (for example, position 6; m6A, position 1; m1A) or cytosine (for example, position 5; m5C, position 3; m3C) which is typically methylated. Demethylation can be specified using a technique known in the art, and can be measured using, for example, a bisulfite method.

In the present specification, the "cell population" is a population comprising two or more cells, and for example, may be in a state where cells are gathered in a planar manner, or may be a cell mass configured by three-dimensionally adhering cells to each other. The "cell population" may be formed by a single type of cell or may comprise a plurality of types of cells.

In the present specification, "stimulation" means stimulation via TCR. For example, stimulation of T cells includes stimulation using an anti-CD3 antibody and a complex containing the same, stimulation using a peptide bonded to TCR and a complex containing the peptide, stimulation using an antigen-presenting cell, stimulation using an anti-CD3 antibody and an antigen-presenting cell simultaneously, stimulation using a peptide, a protein, and an antigen-presenting cell simultaneously, and the like.

In the present specification, "dormant culturing" refers to culturing cells in a state where there is no stimulus as described above.

### (Preferred embodiments)

Preferred embodiments of the present disclosure will be described below. It is understood that the embodiments provided hereinafter are provided for the better understanding of the present disclosure, so that the scope of the present disclosure should not be limited by the following descriptions. Thus, it is apparent that those skilled in the art can refer to the descriptions in the present specification to make appropriate modifications within the scope of the present disclosure. The following embodiments of the present disclosure can be used individually or as a combination.

### (Inducible regulatory T cell)

The present disclosure relates to a high-functional stable inducible regulatory T cell (also referred to as high-functional stable iTreg, HSF iTreg) and use thereof.

In one aspect of the present disclosure, there is provided an inducible regulatory human T cell having at least one feature selected from the group consisting of FoxP3 positivity, CTLA4 positivity, NT5E positivity, ITGAE (CD103) positivity, AREG positivity, CD172g positivity, and CD26 positivity. In one embodiment of the present disclosure, the inducible regulatory T cell of the present disclosure can also have at least two features selected from the group consisting of CTLA4 positivity, NT5E positivity, ITGAE (CD103) positivity, and AREG positivity. Also in one embodiment of the present disclosure, the inducible regulatory T cell of the present disclosure may be at least CTLA4-positive. Although not intended to be limiting, the inducible regulatory T cell of the present disclosure may be CD4-positive or CD8-positive.

In one embodiment of the present disclosure, the inducible regulatory T cell of the present disclosure may be at least CTLA4-positive and FoxP3-positive. In one embodiment of the present disclosure, the inducible regulatory T cell of the present disclosure may be at least CD172g-positive and/or CD26-positive.

The present disclosure provides an inducible regulatory human T cell which is CD172g-positive. Since CD172g is a tyrosine kinase-related protein and is involved in cell adhesion of neurons and the like (adhesion of cerebellar neurons, neurite outgrowth and glial cell attachment), it is expected that the inducible regulatory human T cell which is CD172g-positive has activated functions related to adhesiveness and the like, and has a high effect on various diseases related thereto.

The present disclosure provides an inducible regulatory human T cell which is CD26-positive. Since CD26 is a gene also called DPP4 and is associated with carbohydrate metabolism, insulin metabolism, and immune function (remarkable also in infectious diseases), it is expected that the inducible regulatory human T cell which is CD26-positive has a high effect on various diseases related thereto.

The present disclosure provides an inducible regulatory human T cell which is NT5E-positive. NT5E (CD73) is a cell membrane protein having catalytic action that converts extracellular nucleotides to membrane-permeable nucleosides. The encoded protein is used as a determinant of lymphocyte differentiation. Since defects in this gene may cause calcification of joints and arteries, it is expected that the inducible regulatory human T cell which is NT5E-positive has a high effect on various diseases related thereto.

The present disclosure provides an inducible regulatory human T cell which is ITGAE (CD103)-positive. ITGAE (CD103) encodes an α-integrin with an I domain and undergoes post-translational cleavage in the extracellular domain to generate disulfidelinked heavy and light chains. This protein binds a β7-integrin to form E-cadherin binding integrin known as the human mucosal lymphocyte-1 antigen. This protein is preferentially expressed in human intestinal intraepithelial lymphocytes (IEL), and may function as an accessory molecule for IEL activation, in addition to its role of adhesion. Therefore, it is expected that the inducible regulatory human T cell which is ITGAE (CD103)-positive has a high effect on various diseases related thereto.

The present disclosure provides an inducible regulatory human T cell which is AREG-positive. AREG is a member of the epidermal growth factor family and is associated with the epidermal growth factor (EGF) and the transforming growth factor α (TGF-α). This protein interacts with the EGF/TGF-α receptor to promote normal epithelial cell growth and inhibit the growth of certain aggressive cancer cell lines. This protein also functions in the development of mammary gland, ovum and bone tissue. This gene is associated with a skin phenotype similar to psoriasis and is also associated with other pathological diseases including various types of cancer and inflammatory conditions. Therefore, it is expected that the inducible regulatory human T cell which is AREG-positive has a high effect on various diseases related thereto.

The present disclosure provides an inducible regulatory human T cell which is CTLA4-positive. CTLA4 is a protein that belongs to the immunoglobulin superfamily and transmits a suppressive signal to T cells. The membrane-bound CTLA4 functions as a homodimer linked by a disulfide bond, and the soluble CTLA4 functions as a monomer. Mutations in this gene are said to be associated with insulin-dependent diabetes, Basedow's disease, Hashimoto's thyroiditis, celiac disease, systemic lupus erythematosus, thyroidassociated orbital disease, and other autoimmune diseases. Therefore, it is expected that the inducible regulatory human T cell which is CTLA4-positive has a high effect on various diseases related thereto.

The expression of FoxP3 in the regulatory T cell can be induced in a test tube by culturing CD4-positive T cells in a medium containing IL2 and TGFβ in the presence of an anti-CD3 antibody and an anti-CD28 antibody, and then culturing the cells in a medium containing IL2 and TGFβ. Although some methods for inducing an inducible regulatory T cell from a peripheral T cell are known, the expression of FoxP3 in the inducible regulatory T cell is unstable, and the expression of many functional molecules other than FoxP3 has not been confirmed.

In recent years, culturing methods for causing DNA demethylation induction have been developed by a method in which ascorbic acid is added to a medium in an inducible regulatory T cell (Kasahara et al. Int. Immunol. (2017) 29(10): 457-469) and a method in which CD28 antibody stimulation is not used (Mikami et al. Proc Natl Acad Sci USA. (2020) 117(22): 12258-12268.). However, the expression of functional molecules has not been confirmed even in the inducible regulatory T cell prepared by this method, and sufficient immunosuppressive activity has not been obtained. Also in clinical trials, one clinical trial using the inducible regulatory T cell has been conducted for GVHD, but the proportion of inducible regulatory T cells used is low, and efficacy such as prevention of GVHD has not been recognized at present (MacMillan et al., Blood Adv. (2021) 5(5): 1425-1436).

In the present disclosure, an inducible regulatory T cell in which the expression of FoxP3 is stable, and advantageously, an immunosuppressive effect is stably maintained can be provided, and such an inducible regulatory T cell can be prepared, for example, by a method for preparing an inducible regulatory T cell as described elsewhere in the specification of the present application. For example, the present disclosure can provide an inducible regulatory T cell obtained by a method including the steps of: (a) stimulating a CD4-positive T cell or a CD8-positive T cell in peripheral blood in a first basal medium for about 1 to about 5 days; (b) dormant culturing the cell obtained in step (a) in a medium containing IL-2 for at least about 1 to about 3 days; (c) stimulating the cell obtained in step (b) in a second basal medium for about 1 to about 5 days; and (d) dormant culturing the cell obtained in step (c) in a medium containing IL-2 for at least about 1 to about 3 days.

The inducible regulatory T cell prepared in the present disclosure has an inducible regulatory T cell specific demethylation state. The fact that the obtained regulatory T cell is in a regulatory T cell-specific demethylation state can be confirmed, for example, by the fact that the CNS2 site of the FoxP3 gene (FOXP3 (all in italics)) is demethylated. Since such a demethylation state can be an index of a stable type, the inducible regulatory T cell of the present disclosure can be shown to be a stable inducible regulatory T cell by confirming the demethylation state.

In the present specification, the immunosuppressive activity or immunosuppressive effect of the inducible regulatory T cell of the present disclosure can be confirmed, for example, by measuring Cell Trace Violet intensity in responder T cells. The inducible regulatory T cell of the present disclosure can stably provide an immunosuppressive activity or immunosuppressive effect, and for example, the inducible regulatory T cell of the present disclosure can provide an immunosuppressive activity or immunosuppressive effect for at least about 2 weeks. As shown in Examples below, the inducible regulatory T cell of the present disclosure can have a higher immunosuppressive effect than conventional regulatory T cells (including inducible and naturally occurring regulatory T cells). Therefore, the inducible regulatory T cell of the present disclosure can also be referred to as a functional or high-functional inducible regulatory T cell.

In one embodiment, the inducible regulatory T cell of the present disclosure can stably express FoxP3. Therefore, the inducible regulatory T cell of the present disclosure can also be referred to as a stable inducible regulatory T cell. In one aspect, the inducible regulatory T cell of the present disclosure is highly functional and stable, and can be referred to as a high-functional stable inducible regulatory T cell (HSF iTreg).

In one embodiment of the present disclosure, whether or not a marker in the inducible regulatory T cell of the present disclosure is positive can be determined by positive rate measurement with a flow cytometry. For example, analysis is performed with a flow cytometer, and it is possible to determine whether or not cells are positive from the proportion of cells showing an antigen expression level equal to or higher than a reference level. It can also be classified into negative, weakly positive, medium positive, strongly positive (weakly positive, medium positive, and strongly positive are collectively referred to as "positive"), and the like according to the expression intensity of the cell surface marker. For example, depending on the setting of the instrument, when values of the median fluorescence intensity of each marker/the median fluorescence intensity of the negative control (staining with an isotype control antibody) is less than 5, 5 or more and less than 10, 10 or more and less than 30, and 30 or more, the values can be determined as negative, weakly positive, medium positive, and strongly positive, respectively. Such determinations can be made as exemplified in (positive rate measurement by flow cytometry), but the present disclosure is not limited thereto.

In one embodiment of the present disclosure, the expression intensity of a cell surface marker in the inducible regulatory T cell of the present disclosure may be determined, for example, based on the result of analysis by a BD FACSLyric flow cytometer (BD Biosciences) using a sample prepared as described in Examples of the present application, and expression intensities of 10² or more, 10³ or more, and 10⁴ or more may be determined as weakly positive, medium positive, and strongly positive, respectively, or an expression intensity of 10³ or more may be determined as strongly positive and an expression intensity of 10³ or less may be determined as weakly positive, or an expression intensity of 10² or more may be determined as strongly positive and an expression intensity of 10² or less may be determined as weakly positive. Such determination can be appropriately set according to experimental conditions, experimental purposes, cell types, device setting conditions, and the like, and the present disclosure is not limited thereto. Also in the case of analysis with a flow cytometer device other than the BD FACSLyric flow cytometer, the expression intensity in another device corresponding to the expression intensity in the case of analysis with the BD FACSLyric flow cytometer can be calculated, and a value indicating the expression intensity can be appropriately derived according to the device to be used.

In one embodiment of the present disclosure, the inducible regulatory T cell prepared in the present disclosure can be induced from any cell, but preferably can be induced from a human peripheral blood T cell or a human tissue-derived T cell.

In one embodiment of the present disclosure, the inducible regulatory T cell or the cell population thereof of the present disclosure can be directed to human cells, and can be an inducible regulatory human T cell or a cell population thereof induced by a predetermined technique using a T cell obtained from human peripheral blood as a material. The properties of human cells and mouse cells are clearly different, and even if the same cell surface marker is present, the properties of the cells cannot be considered to be the same. For example, in the field of inducible regulatory T cells, the techniques that can be applied to mice and humans are different, and in the case of mice, most of lymphocytes are unsensitized with antigen, whereas in the case of humans, T cells in peripheral blood have various degrees of antigen sensitization and activation. Therefore, in the case of mice, it is possible to directly prepare a high-functional stable inducible regulatory T cell from a T cell collected from a mouse lymphatic tissue, but in the case of humans, such direct preparation is difficult, and it is not possible to regard a cell as a similar cell simply by the presence of a cell surface marker.

In one aspect of the present disclosure, there is provided a cell population comprising the inducible regulatory human T cell as described above, wherein the cell population has a proportion of at least one feature selected from the group consisting of FoxP3 positivity, CTLA4 positivity, NT5E positivity, ITGAE (CD103) positivity, AREG positivity, CD 172g positivity, and CD26 positivity of about 50% or more.

In another aspect of the present disclosure, there is provided a cell population comprising an inducible regulatory human T cell, wherein the cell population has a proportion of each of CTLA4 positivity and FoxP3 positivity of about 50% or more. In one embodiment, the cell population of the present disclosure can have a proportion of CTLA4 positivity and FoxP3 positivity of about 60% or more, about 65% or more, about 70% or more, about 75% or more, about 80% or more, about 85% or more, about 90% or more, about 95% or more, about 97% or more, or about 99% or more.

In one embodiment of the present disclosure, in the cell population of the present disclosure, the proportions of at least CD172g positivity and/or CD26 positivity each can be about 50% or more. In one embodiment, the cell population of the present disclosure can have a proportion of CD172g positivity and/or CD26 positivity of about 60% or more, about 65% or more, about 70% or more, about 75% or more, about 80% or more, about 85% or more, about 90% or more, about 95% or more, about 97% or more, or about 99% or more.

In one embodiment of the present disclosure, in the cell population of the present disclosure, the proportion of FoxP3 positivity can be about 50% or more. In one embodiment, the cell population of the present disclosure can have a proportion of FoxP3 strong positivity of about 60% or more, about 65% or more, about 70% or more, about 75% or more, about 80% or more, about 85% or more, about 90% or more, about 95% or more, about 97% or more, or about 99% or more. Although not intended to be limiting, in such a cell population, the measurement of the expression intensity of the cell surface marker can be performed by the positive rate measurement with a flow cytometry as described above.

In another aspect of the present disclosure, there is provided a cell population comprising an inducible regulatory human T cell, wherein the cell population has a proportion of FoxP3 positivity of about 50% or more. Although not intended to be limiting, in one embodiment, such a cell population can comprise FoxP3-positive inducible regulatory human T cells of about 50% or more, about 60% or more, about 65% or more, about 70% or more, about 75% or more, about 80% or more, about 85% or more, about 90% or more, about 95% or more, about 97% or more, or about 99% or more, on a cell count basis.

In another aspect of the present disclosure, there is provided a cell population comprising an inducible regulatory human T cell, wherein the cell population has a proportion of CTLA4 positivity of about 50% or more. Although not intended to be limiting, in one embodiment, such a cell population can comprise CTLA4-positive inducible regulatory human T cells of about 50% or more, about 60% or more, about 65% or more, about 70% or more, about 75% or more, about 80% or more, about 85% or more, about 90% or more, about 95% or more, about 97% or more, or about 99% or more, on a cell count basis.

In another aspect of the present disclosure, there is provided a cell population comprising an inducible regulatory human T cell, wherein the cell population has a proportion of NT5E positivity of about 50% or more. Although not intended to be limiting, in one embodiment, such a cell population can comprise NT5E-positive inducible regulatory human T cells of about 50% or more, about 60% or more, about 65% or more, about 70% or more, about 75% or more, about 80% or more, about 85% or more, about 90% or more, about 95% or more, about 97% or more, or about 99% or more, on a cell count basis.

In another aspect of the present disclosure, there is provided a cell population comprising an inducible regulatory human T cell, wherein the cell population has a proportion of ITGAE (CD103) positivity of about 50% or more. Although not intended to be limiting, in one embodiment, such a cell population can comprise ITGAE (CD103)-positive inducible regulatory human T cells of about 50% or more, about 60% or more, about 65% or more, about 70% or more, about 75% or more, about 80% or more, about 85% or more, about 90% or more, about 95% or more, about 97% or more, or about 99% or more, on a cell count basis.

In another aspect of the present disclosure, there is provided a cell population comprising an inducible regulatory human T cell, wherein the cell population has a proportion of AREG positivity of about 50% or more. Although not intended to be limiting, in one embodiment, such a cell population can comprise AREG-positive inducible regulatory human T cells of about 50% or more, about 60% or more, about 65% or more, about 70% or more, about 75% or more, about 80% or more, about 85% or more, about 90% or more, about 95% or more, about 97% or more, or about 99% or more, on a cell count basis.

In another aspect of the present disclosure, there is provided a cell population comprising an inducible regulatory human T cell, wherein the cell population has a proportion of CD172g positivity of about 50% or more. Although not intended to be limiting, in one embodiment, such a cell population can comprise CD172g-positive inducible regulatory human T cells of about 50% or more, about 60% or more, about 65% or more, about 70% or more, about 75% or more, about 80% or more, about 85% or more, about 90% or more, about 95% or more, about 97% or more, or about 99% or more, on a cell count basis.

In another aspect of the present disclosure, there is provided a cell population comprising an inducible regulatory human T cell, wherein the cell population has a proportion of CD26 positivity of about 50% or more. Although not intended to be limiting, in one embodiment, such a cell population can comprise CD26-positive inducible regulatory human T cells of about 50% or more, about 60% or more, about 65% or more, about 70% or more, about 75% or more, about 80% or more, about 85% or more, about 90% or more, about 95% or more, about 97% or more, or about 99% or more, on a cell count basis.

As described above, since the properties of human cells and mouse cells are clearly different from each other, even if the conditions for induction are the same, the ratio of induction of specific cells is different between mouse cells and human cells, and in the present disclosure, when induction is performed by a predetermined induction method, a cell population having a proportion of positivity for a predetermined cell surface marker of about 50% or more can be obtained, and preferably, a cell population "having a proportion of each of CTLA4 positivity and FoxP3 positivity of about 50% or more" can be obtained.

CTLA4 is a molecule localized in a cell, and is not at least at a level that can be used as an index of concentration or purification. Since FoxP3 is a transcription factor and is a molecule existing in cells, FoxP3 cannot be detected at all on the surface and cannot be used for purification. That is, since CTLA4 and FoxP3 are markers expressed "in" T cells, a cell population having a proportion of CTLA4 positivity and/or FoxP3 positivity of about 50% or more cannot be obtained by being concentrated while sorting CTLA4 and/or FoxP3 as an index. Therefore, a cell population having a proportion of each of CTLA4 positivity and FoxP3 positivity of about less than 50% is obtained according to the conventional method, such a cell population is used as a raw material and concentrated using CTLA4 and/or FoxP3 as an index, and a cell population having a proportion of each of CTLA4 positivity and FoxP3 positivity in the cells of about 50% or more cannot also be obtained.

In one embodiment of the present disclosure, the inducible regulatory human T cell of the invention of the present application is mainly responsible for the immunosuppressive function by Treg, and when this cell accounts for more than half in a certain cell population, a medically effective immunosuppressive effect can be stably achieved, which is also important for technical and medical effects. That is, since a stable cell preparation can be provided by containing 50% or more of T cells exhibiting a medically effective immunosuppressive effect, this effect is an extremely important point in medicine, and is an important point not only in the difference in amount or numerical value but also as a problem of quality of whether the cell preparation is established as a preparation.

In one embodiment of the present disclosure, in the cell population of the present disclosure, the inducible regulatory human T cell can further include features of ITGAE (CD103) positivity, NT5E positivity, and/or AREG positivity. These cell markers are genes that play an important role in immunosuppressive function, and since these markers are highly expressed, it is possible to maintain more functional stability after administration of cells as compared with conventional inducible regulatory T cells. For example, CD103 is important for migration of a regulatory T cell to an inflammation site, NT5E (CD73) is important for production of adenosine having an immunosuppressive ability, and AREG is important for regeneration of tissue by a regulatory T cell. The cell population of the present disclosure can achieve the effect that functional stability can be maintained due to high expression of these markers.

In one embodiment of the present disclosure, the T cell in the cell population of the present disclosure can be a regulatory T cell. In this case, about 60% or more, about 65% or more, about 70% or more, about 75% or more, about 80% or more, about 85% or more, about 90% or more, about 95% or more, about 97% or more, or about 99% or more of the regulatory T cells of the cell population of the present disclosure can be the inducible regulatory T cell as described elsewhere in the specification of the present application.

In one embodiment of the present disclosure, the cell population of the present disclosure may comprise cells other than the T cells, but preferably about 60% or more, about 65% or more, about 70% or more, about 75% or more, about 80% or more, about 85% or more, about 90% or more, about 95% or more, about 97% or more, or about 99% or more of the cell population of the present disclosure may be T cells, and preferably about 90% or more may be T cells.

In one aspect of the present disclosure, there is provided a pharmaceutical composition comprising the inducible regulatory T cell or cell population of the present disclosure. In another aspect, there is provided a regenerative medical material or product comprising the inducible regulatory T cell or cell population of the present disclosure. This pharmaceutical composition or regenerative medical material or product can be used in an autoimmune disease, an inflammatory disease, and an allergy, which can be treated by an immunosuppressive effect. These pharmaceutical and regenerative medical material or product can be used with media or any other additive used in the art. As such a medium, a medium obtained by adding a necessary factor to a basal medium for culturing animal cells used for culturing cells can be used. Examples of such a medium are detailed elsewhere in the present specification. Examples of components added to the medium are also detailed elsewhere in the present specification. In the case of providing a medium as such a product, DMSO or the like may be contained.

### (Preparation method)

The present disclosure provides a method for preparing the inducible regulatory T cell of the present disclosure. The method of the present disclosure is a novel method capable of preparing a highly functional and stable inducible regulatory T cell and will be described in detail below in the present specification.

In one aspect of the present disclosure, there is provided a method for preparing an inducible regulatory T cell, the method including the steps of: (a) stimulating a CD4-positive T cell or a CD8-positive T cell in peripheral blood in a first basal medium for about 1 to about 5 days; (b) dormant culturing the cell obtained in step (a) in a medium containing IL-2 for at least about 1 to about 3 days; (c) stimulating the cell obtained in step (b) in a second basal medium for about 1 to about 5 days; and (d) dormant culturing the cell obtained in step (c) in a medium containing IL-2 for at least about 1 to about 3 days. In the present disclosure, the inducible regulatory T cell of the present disclosure can be prepared using any of a CD4-positive T cell and a CD8-positive T cell as a raw material, and in one embodiment, the inducible regulatory T cell of the present disclosure can also be prepared using mixed cells of a CD4-positive T cell and a CD8-positive T cell as a raw material.

In one embodiment, the method of the present disclosure can also be a method of preparing a regulatory T cell from a human peripheral T cell (including a CD4-positive T cell or a CD8-positive T cell), the method including a step of culturing a human peripheral T cell in a medium containing TGFβ and IL-2 in the presence of anti-CD3 antibody stimulation, a step of culturing the cell in a medium containing IL-2 in the absence of an anti-CD3 antibody, and a step of culturing the cell again in a medium containing TGFβ and IL-2 in the presence of anti-CD3 antibody stimulation.

In one embodiment, stimulation of T cells is nor particularly limited as long as it is stimulation in obtaining of Treg and stimulation via TCR. For example, stimulation of T cells can include stimulation using an anti-CD3 antibody and a complex containing the same, stimulation using a peptide bonded to TCR and a complex containing the peptide, stimulation using an antigen-presenting cell, stimulation using an anti-CD3 antibody and an antigen-presenting cell simultaneously, stimulation using a peptide, a protein, and an antigen-presenting cell simultaneously, and the like, but a medium and conditions thereof are not particularly limited as long as Treg can be obtained.

In one embodiment, a medium and conditions for the dormant culturing are not particularly limited as long as the cells are cultured in a state without stimulation as described above.

In the present specification, the "anti-CD3 antibody stimulation" means that stimulation specific to a CD3 receptor on a cell is provided. Examples of the CD3 stimulation include an anti-CD3 agonistic antibody. As the anti-CD3 agonistic antibody, a commercially available product as a research reagent may be used, or an anti-CD3 agonistic antibody may be prepared by a conventional method. As the anti-CD3 antibody, for example, those derived from animals such as a mouse, a rabbit, a goat, or a cow and derived from a human can be used.

In one embodiment, in the method of the present disclosure, an inducible regulatory human T cell having functionality and being stable can be obtained by once inducing iTreg (stimulating a T cell causing demethylation induction) (step a), recovering the cell by replacing a medium and performing dormant culturing for 1 to 3 days (step b), and further stimulating the T cell once to cause demethylation induction (step c). It is generally common technical knowledge that the T cell exhibits apoptosis when stimulated twice, but in the method of the present disclosure, it has been found that by performing "dormant culturing" and "second stimulation", an inducible regulatory human T cell having functionality and being stable can be obtained without exhibiting apoptosis.

That is, in the method of the present disclosure, since an inducible regulatory human T cell having functionality and being stable can be obtained by performing T cell stimulation, dormant culturing, and then re-stimulation, a desired effect of the present disclosure can be achieved as long as the inducible regulatory human T cell obtained in this manner or a cell population comprising such an inducible regulatory human T cell is obtained. Therefore, in one embodiment of the present disclosure, the types of a medium for culture and stimulation are not particularly limited, and by using a medium having an arbitrary composition and an arbitrary type of stimulation, an inducible regulatory human T cell having functionality and being stable can be obtained.

In one embodiment, the medium used in each step may further contain retinoic acid and/or ascorbic acid. Preferably, the medium can contain ascorbic acid. In one embodiment, the medium may further contain a CDK8 inhibitor, a CDK19 inhibitor, and/or a CDK8/19 inhibitor. Preferably, the medium can contain a CDK8 inhibitor, a CDK19 inhibitor, and/or a CDK8/19 inhibitor.

In one embodiment of the present disclosure, the first basal medium and the second basal medium can each independently contain at least one, at least two, at least three, at least four, at least five, or all factors selected from the group consisting of an anti-CD3 antibody, TGF-β1, IL-2, retinoic acid, a CDK8 inhibitor, a CDK19 inhibitor, a CDK8/19 inhibitor, and ascorbic acid. In another embodiment, the first basal medium and the second basal medium can also each independently contain anti-CD3 antibody, TGF-β1, IL-2, retinoic acid, a CDK8 inhibitor, a CDK19 inhibitor, a CDK8/19 inhibitor, and ascorbic acid. The concentration of the factor contained as each of these components can be a normal concentration used in the art. In one embodiment, the concentration of a CDK8 inhibitor, a CDK19 inhibitor, and/or a CDK8/19 inhibitor that may be used may be any suitable concentration that may be used in the art, for example, in the case of using Senexin A, the concentration can be about 0.1 µM or more, about 0.5 µM or more, about 1 µM or more, about 2 µM or more, about 3 µM or more, about 4 µM or more, about 5 µM or more, about 6 µM or more, about 7 µM or more, about 8 µM or more, about 9 µM or more, about 10 µM or more, about 12 µM or more, about 14 µM or more, about 16 µM or more, about 18 µM or more, about 20 µM or more, or the like, but is not limited to these concentrations, and can be appropriately changed by those skilled in the art according to other medium compositions.

In one embodiment, in the method of the present disclosure, a regulatory T cell is prepared from a human peripheral T cell. The peripheral T cells include naive regulatory T cells, CD4-positive T cells, CD8-positive T cells, and the like. The regulatory T cell may be induced from a culture containing a plurality of types of T cells, or the regulatory T cell may be induced after specific cells such as CD4-positive T cells and CD8-positive T cells are isolated from these cells. The regulatory T cell may be induced after a T cell specific to a specific antigen is isolated. Thus, the inducible regulatory T cell of the present disclosure includes a CD4-positive inducible regulatory T cell and a CD8-positive inducible regulatory T cell. In the present specification, the term "CD4-positive" or "CD4+" refers to a CD4-positive CD8-negative single positive cell unless otherwise specified. In the preparation method in the present disclosure, either CD4-positive or CD8-positive T cell can be used as a raw material, and even after generation of the inducible regulatory T cell, the CD4-positive or CD8-positive feature can be maintained unless special manipulation is performed.

In one embodiment, the antibody in the method of the present disclosure may be added to the medium, or may be immobilized on the inner wall of a culture vessel or the surface of an insoluble carrier. As the insoluble carrier, a member capable of physically or chemically binding the anti-CD3 antibody and insoluble in an aqueous solution can be used. Examples of the material capable of physically adsorbing the anti-CD3 antibody include synthetic resins such as polystyrene, polyethylene terephthalate, polycarbonate, and polypropylene, and glass. The shape of the insoluble carrier is not particularly limited, and for example, a plate shape, a bead shape, a container shape, or the like can be adopted. The amount of the anti-CD3 antibody varies depending on the titer and origin of an antibody to be used, but may be appropriately set so as to provide sufficient stimulation for induction of the regulatory T cell.

In one embodiment, in the method of the present disclosure, a medium obtained by adding a necessary factor to a basal medium for culturing animal cells can be used for cell culture. Examples of the basal medium for culturing animal cells that can be used in the method of the present disclosure include Iscove's modified Eagle's Medium, Ham's F12 medium, MEM Zinc Option medium, IMEM Zinc Option medium, IMDM medium, Medium 199 medium, Eagle's Minimum Essential Medium (EMEM), αMEM medium, Dulbecco's modified Eagle's Medium (DMEM), RPMI 1640 medium, Fischer's medium, and a mixed medium of these or a medium in which a part of the composition of the foregoing medium is modified.

The basal medium may contain serum (for example, fetal bovine serum (FBS)) or may be serum-free. The serum-free medium may optionally contain, for example, one or more serum alternatives such as albumin, bovine serum albumin (BSA), transferrin, apotransferrin, KnockOut Serum Replacement (KSR) (serum alternative in ES cell culture) (Thermo Fisher Scientific), N2 supplement (Thermo Fisher Scientific), B27 supplement (Thermo Fisher Scientific), fatty acid, insulin, a collagen precursor, trace elements, 2-mercaptoethanol, 3'-thiol glycerol, and monothioglycerol. The basal medium may also contain one or more substances such as lipids (for example, chemically defined lipid concentrate), amino acids, L-glutamine, GlutaMAX (Thermo Fisher Scientific), nonessential amino acids (NEAA), vitamins (for example, nicotinamide and ascorbic acid), growth factors, antibiotics (for example, penicillin and streptomycin), antioxidants, pyruvate, buffers, inorganic salts, and equivalents thereof.

In one embodiment, as the basal medium, RPMI 1640 medium containing serum and HEPES (4-(2-hydroxyethyl)-1-piperazineethanesulfonic acid) is exemplified.

In the method of the present disclosure, the cells may be cultured under general animal cell culture conditions. The culture temperature is, but is not limited to, about 30 to 40°C and preferably about 37°C. The culturing is preferably carried out in an atmosphere of CO₂-containing air, and the CO₂ concentration is preferably about 2 to 5%.

In the method of the present disclosure, the anti-CD3 antibody may be added to the medium as it is, or may be immobilized on the inner wall of a culture vessel or the surface of an insoluble carrier. The amount of the anti-CD3 antibody varies depending on the titer and origin of an antibody to be used, but may be appropriately set so as to provide sufficient stimulation for induction of the regulatory T cell.

As TGFβ to be used, TGFβ1, TGFβ2, and TGFβ3 are exemplified, and for example, TGFβ1 is used. The concentration of TGFβ may be appropriately determined by those skilled in the art, and is not particularly limited. In the case of using TGFβ1 or TGFβ3 as TGFβ, the concentration thereof in the medium is not particularly limited, but may be set to 0.25 to 25 ng/mL, for example, about 10 ng/mL.

The concentration of IL-2 in the medium used is not limited, and can be set to about 5 U/mLto about 500 U/mL, for example, about 100 U/mL.

Retinoic acid and/or ascorbic acid may be further added to the medium used in the present disclosure. Preferably, the medium contains ascorbic acid. The concentration of ascorbic acid is not limited, but is about 1 to about 100 µg/mL, for example, about 10 µg/mL.

A CDK8 inhibitor, a CDK19 inhibitor, and/or a CDK8/19 inhibitor may be further added to the medium used in the present disclosure. As the CDK8 inhibitor, the CDK19 inhibitor, and/or the CDK8/19 inhibitor, any one can be used, and examples thereof include 4-[1-(2-methyl-1H-benzimidazole-5-yl)-1H-imidazo[4,5-c]pyridine-2-yl]-1,2,5-oxadiazole-3-amine, 3-{ 1-[1-(4-methoxyphenyl)piperidin-4-yl]-4-methyl-1H-imidazo[4,5-c]pyridine-2-yl}pyrazine-2-amine, salts, hydrates, solvates, and the like thereof, and compounds described in US 8598344, WO2013/001310, WO2013/040153, WO2013/116786, WO2014/029726, WO2014/063778, WO2014/072435, WO2014/090692, WO2014/106606, WO2014/123900, WO2014/154723, WO2014/194245, WO2015/049325, WO2015/100420, WO2015/144290, WO2015/159937, WO2015/159938, WO2016/009076, WO2018/139660, or the like. As one example, Senexin A or AS2863619 is exemplified, but the present disclosure is not limited thereto. The concentration of the CDK8 inhibitor, the CDK19 inhibitor, and/or the CDK8/19 inhibitor that can be used may be any suitable concentration that can be used in the art, and may be any suitable concentration described in the above literature, and appropriately can be changed according to other medium compositions by those skilled in the art.

In the method of the present disclosure, a human peripheral T cell is stimulated with an anti-CD3 antibody in a medium containing TGFβ and IL-2 to induce a regulatory T cell, and after performing dormant culturing the cell in an IL-2-containing medium not containing an anti-CD3 antibody, the human peripheral T cell is stimulated again with an anti-CD3 antibody, whereby a regulatory T cell having a high immunosuppressive function can be induced. It can be confirmed that the obtained inducible regulatory T cell has a high immunosuppressive function, for example, by comprehensive gene expression analysis by an RNA sequencing method and a cell proliferation suppression test in vitro.

In one embodiment of the present disclosure, the number of days of culture in step (a) of stimulating a CD4-positive T cell or a CD8-positive T cell in peripheral blood in a first basal medium for about 1 to about 5 days can be appropriately set by those skilled in the art, and is not particularly limited, but can be also set to, for example, about 3 days.

Also in one embodiment, the number of days of culture in the dormant culturing step in step (b) can be appropriately set by those skilled in the art, and is not particularly limited, but can be also set to, for example, about 2 days.

Also in one embodiment, the number of days of culture in the culturing step in the second basal medium in step (c) can be appropriately set by those skilled in the art, and is not particularly limited, but can be also set to, for example, about 3 days.

Also in one embodiment, the number of days of culture in the dormant culturing step in step (d) can be appropriately set by those skilled in the art, and is not particularly limited, but can be also set to, for example, about 2 days. In one embodiment, a regulatory T cell having an induced regulatory T cell specific demethylation state can be proliferated by culturing in an unstimulated state in the presence of IL-2. A stable regulatory T cell culture of a regulatory T cell induced by the fact that the medium may further contain ascorbic acid and by culturing in a medium further containing IL-2 can be obtained.

In order to isolate the regulatory T cell from the obtained cell culture containing the regulatory T cell, the cell may be isolated by a conventional method based on a cell surface marker unique to the regulatory T cell, and for example, the FoxP3-positive fraction may be taken out by a cell sorter. A regulatory T cell having specific antigenic properties may also be isolated as desired.

The inducible regulatory T cell obtained by the method of the present disclosure is expected to be used for the treatment of human inflammatory diseases, for example, autoimmune diseases and allergies.

### (Positive rate measurement by flow cytometry)

In one embodiment, the positive rate measurement with a flow cytometry can be performed as follows.

### Preparation

- Fixation/Permeabilization Concentrate (hereinafter, Buffer) (eBio Science, 00-5123-43)
- Fixation/Permeabilization Diluent (hereinafter, Diluent) (eBio Science, 00-5223-56)
- Permeabilization Buffer (10×) (eBio Science, 00-833-56)
- FOXP3 Monoclonal Antibody (236A/E7), PE (hereinafter, anti-FOXP3 antibody) (eBio Science, 12-4777-42)
- Mouse IgG1 kappa Isotype Control (P3.6.2.8.1), PE (hereinafter, PE control)

### (eBio Science)

- BV421, Mouse, Anti-Human, CD152 (hereinafter, anti-CTLA4 antibody) (BD)
- BV421 Mouse IgG2a, k Isotype Control (hereinafter, BV421 control) (BD)
- CD4 Monoclonal Antibody (RPA-T4), APC (hereinafter, anti-CD4 antibody)

### (eBio Science)

- Mouse IgG1 kappa Isotype Control (P3.6.2.8.1), APC (hereinafter, APC control)

### (eBio Science)

- D-PBS (Nacalai Tesque, 14249-95)
- FBS (HyClone, SH30084.03)
- 0.5 mol/l-EDTA solution (pH 8.0) (Nacalai Tesque, 06894-14)
- MilliQ water
- Centrifuge
- Safety cabinet
- Micropipette (P200, P1000)
- 5 mL polystyrene round tube (hereinafter, dedicated tube) (Falcon, 352008)
- Nylon mesh (65 µm) (Kyoshin Riko, PP-65N)

### Reagent preparation

• Fixation Buffer (100 µL used per sample)
Buffer and Diluent are mixed at a ratio of 1 : 3.
* Perm Buffer
Permeabilization Buffer (10×) is diluted 10-fold with MilliQ water.
* FACS Bufer (in the case of 500 mL preparation)

| | |
|---|---|
| D-PBS | 489 mL |
| FBS | 10 mL (final concentration: 2%) |
| 0.5 mol/l EDTA solution | 1 mL (final concentration: 1 mM) |

The above reagents are stored at 4°C or on ice after preparation.

### Method

(1) To a 1.5 mL tube, 500 µL of FACS Bufer is added.
(2) To the tube of (1), 1 × 10⁶ final products are added, and gently suspended with a micropipette.
(3) The mixture is centrifuged at 500 × g and 4°C for 5 minutes with a centrifuge.
(4) After removing the supernatant with an aspirator, 100 µL of Fixation Buffer is added and pipetted gently. → Be careful not to whip.
(5) The resultant product is fixed by standing for 30 minutes or more with light shielding on ice. → The time for this may be 45 minutes.
(6) After fixation, 1 mL of Perm Buffer is added to the tube and pipetted gently.
(7) A new 1.5 mL tube is prepared for the number of samples.
(8) Samples are dispensed in 500 µL portions for control and antibody staining.
(9) The mixture is centrifuged at 500 × g and 4°C for 5 minutes with a centrifuge.
(10) An anti-FOXP3 antibody (stock solution concentration = 0.05 mg/ml), an anti-CTLA4 antibody (Lot: 0030269 stock solution concentration = 0.2 mg/ml), an anti-CD4 antibody (stock solution concentration = 0.1 mg/ml) are prepared in 100-fold dilution with Perm Buffer during centrifugation (hereinafter, referred to as an antibody preparation solution). For control samples, PE control (stock solution concentration = 0.1 mg/ml), BV421 control (stock solution concentration = 0.2 mg/ml), and APC control (stock solution concentration = 0.1 mg/ml) are adjusted to the same concentration as the antibody of the corresponding dye (hereinafter, referred to as a control solution).
   * CTLA4 staining is not performed in the in-process control test.
(11) After removing the supernatant with an aspirator, 100 µL of an antibody preparation solution is added to an antibody-stained sample and 100 µL of the control solution is added to a control sample, and pipetting is performed gently. → Be careful not to whip.
(12) The resultant product is fixed by standing for 60 minutes with light shielding on ice.
(13) Startup of a measuring instrument is carried out during dyeing.
(14) After staining, 1 mL of FACS Bufer is added and pipetted gently.
(15) The mixture is centrifuged at 500 × g and 4°C for 5 minutes with a centrifuge. → After removing the supernatant with an aspirator, (14) and (15) are repeated once more and washing is performed.
(16) After removing the supernatant with an aspirator, 500 µL of FACS Bufer is added and pipetted gently.
(17) A nylon mesh is placed on the dedicated tube with tweezers, and the suspension is filtered.
(18) Analysis is performed by any flow cytometer instrument. The number of data collected cells is set to 10000 or more. In calculation of the target antigen positive rate, a reference line is drawn so that the positive rate of the control sample is 5% or less, and the proportion of cells showing an antigen expression level equal to or higher than the reference is taken as the positive rate.

### Remarks

- Reagents used for fixation and staining can be purchased in sets of three as "Foxp3/Transcription Factor Staining Buffer Set" (Cat.:00-5523).
- There is no problem with the device settings and the like as long as they do not deviate to the extent that it can be clearly determined from a general and scientific viewpoint that normal measurement has not been performed. The clear deviation is a case where various signals of the cell population of interest fall below a set fluorescence threshold value, a case where various signal values of a control sample or a sample to be measured fall below or exceed a limit value that can be normally measured by a device, or the like.

### (Immunosuppressive activity measurement)

The cell of the present disclosure may have immunosuppressive activity. The immunosuppressive activity can be measured by various methods.

In one embodiment, as described in Examples, the suppression can be determined by measuring cell proliferation due to an immune reaction caused by responder T cells. In addition, various kits can be used, and a Treg immunosuppressive assay (horizon discovery; https://www.horizondi scoverykk.com/products/research-services/immune-cell-based-assay/immune-suppression-assays/#Treg), and the like can be used, but not limited thereto. The immunosuppressive activity of the cell of the present disclosure may be enhanced over conventional iTreg.

### (General techniques)

The molecular biological method, the biochemical method, and the microbiological method used in the present specification are well known and commonly used in the art, and are disclosed in, for example: Sambrook J. et al. (1989). Molecular Cloning: A Laboratory Manual, Cold Spring Harbor and its 3rd Ed. (2001); Ausubel, F.M. (1987).Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990).PCR Protocols: A Guide to Methods and Applications, Academic Press; Ausubel, F.M. (1992). Short Protocols in Molecular Biology: A Compendium of Methods from Current Protocols in Molecular Biology, Greene Pub. Associates; Ausubel, F.M. (1995).Short Protocols in Molecular Biology: A Compendium of Methods from Current Protocols in Molecular Biology, Greene Pub. Associates; Innis, M.A. et al. (1995).PCR Strategies, Academic Press; Ausubel, F.M. (1999).Short Protocols in Molecular Biology: A Compendium of Methods from Current Protocols in Molecular Biology, Wiley, and annual updates; Sninsky, J.J. et al. (1999). PCR Applications: Protocols for Functional Genomics, Academic Press; separate-volume laboratory medicine "Experimental technique for gene transfer & expression analysis" YODOSHA CO., LTD., 1997; and the like, and parts (or could be all) of these documents related to the present specification are incorporated herein by reference.

For a DNA synthesis technique and nucleic acid chemistry for producing an artificially synthesized gene, for example, gene synthesis and fragment synthesis services such as GeneArt, GenScript, Integrated DNA Technologies (IDT) can also be used, and in addition, the DNA synthesis technique and nucleic acid chemistry are disclosed in, for example: Gait, M.J. (1985). Oligonucleotide Synthesis: A Practical Approach, IRL Press; Gait, M.J. (1990). Oligonucleotide Synthesis: A Practical Approach, IRL Press; Eckstein, F. (1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; Blackburn, G.M. et al. (1996). Nucleic Acids in Chemistry and Biology, Oxford University Press; Hermanson, G.T. (I996). Bioconjugate Techniques, Academic Press; and the like, and parts of these documents related to the present specification are incorporated herein by reference.

In the present specification, "or" is used when "at least one or more" of the elements listed in the sentence can be used. The same applies to "or". In the present specification, when explicitly described as "within a range" of "two values", the two values themselves are included in the range.

Reference literatures such as scientific literatures, patents, and patent applications cited in the present specification are incorporated in the present specification by reference to the same extent that the entirety of each document is specifically described.

As described above, the present disclosure has been described while showing preferred embodiments to facilitate understanding. Hereinafter, the present disclosure is described hereinafter based on Examples, the above descriptions and the following Examples are not provided to limit the present disclosure, but for the sole purpose of exemplification. Thus, the scope of the present disclosure is not limited to the embodiments and Examples specifically described in the present specification and is limited only by the scope of claims.

### Examples

In the following examples, glutamine-free RPMI 1640 (10% FCS (v/v), 60 µg/mL penicillin G, 100 µg/mL streptomycin, containing 10 mM HEPES) was used as a basal medium. If necessary, 30 to 300 mg/L of L-glutamine was added to the medium and used.

### Bisulfite sequence

Genomic DNA was obtained from the induced cells, and after treatment using MethylEasy Xceed Rapid DNA Bisulphite Modification Kit (Human Genetic Signatures), the demethylation of genes characteristic of the regulatory T cell was examined. Analysis of demethylation of each gene used known methods and primers (Floess et al. (2007) PloS Biology Volume 5, Issue 2, e38, and Ohkura et al. (2012) Immunity 37(5) 785-799).

In the analysis of the human Foxp3 CNS2 region, 5'-TTGGGTTAAGTTTGTTGTAGGATAG-3' (SEQ ID NO: 1) was used on the forward side, and 5'-ATCTAAACCCTATTATCACAACCCC-3' (SEQ ID NO: 2) was used on the reverse side.

### Acquisition of fractions of each T cell

Human CD4 +T cells were isolated from PBMCs of healthy individuals. The concentrated PBMCs were purified using CliniMACS CD4 GMP MicroBeads according to the product protocol.

### (Example 1: Preparation of regulatory T cell from CD4-positive human peripheral T cell)

The CD4-positive T cell was stimulated for 3 days in a basal medium containing an anti-CD3 antibody (the antibody having a concentration of 10 µg/ml is added in an amount of 100 µL per well, and is allowed to stand at room temperature for 60 minutes to be immobilized on the container), hIL-2 (100 U/ml), hTGFβ1 (10 ng/ml), retinoic acid (1 µM), and Senexin A (5 µM). Thereafter, the cell was subjected to dormant culturing in a basal medium containing hIL-2 (100 U/ml) for 2 days. After 2 days, the medium was replaced in the same manner, and dormant culturing was further performed for 2 days. After 2 days, the cell was stimulated again for 2 days in a basal medium containing an anti-CD3 antibody (the antibody having a concentration of 10 µg/ml is added in an amount of 100 µL per well, and is allowed to stand at room temperature for 60 minutes to be immobilized on the container), hIL-2 (100 U/ml), hTGFβ1 (10 ng/ml), retinoic acid (1 µM), Senexin A (5 µM), and ascorbic acid (10 µg/ml). Thereafter, the cell was subjected to dormant culturing in a basal medium containing hIL-2 (100 U/ml) for 2 days. After 2 days, the medium was replaced in the same manner, and dormant culturing was further performed for 2 days. The expression of FoxP3, CTLA4, and Helios in the regulatory T cell obtained after 2 days was analyzed by a flow cytometry method (FIG. 1).

Conventional inducible regulatory T cells (conventional iTreg) have insufficient expression of functional molecules (for example, in this case, CTLA4) and low FoxP3 induction efficiency. On the other hand, CTLA4 and FoxP3 are expressed in endogenous regulatory T cells (nTreg), and representative Helios is expressed as an nTreg marker molecule. The highly functional inducible regulatory T cell obtained by the preparation method of the present application is Helios-negative, and can be confirmed to be a population containing many cells expressing FoxP3 and CTLA4. Helios is a marker of a regulatory T cell resident in the body, and a negative Helios indicates that it is an inducible regulatory T cell. Since the inducible regulatory T cell obtained by the conventional method is negative for CTLA4 as an example, it can be seen that the obtained inducible regulatory T cell is an inducible regulatory T cell newly induced in vitro at least in terms of positive for CTLA4.

The demethylation state of cells cultured in the same experimental system was confirmed by a bisulfite sequencing method (FIG. 2). The inducible regulatory T cell obtained by the preparation method of the present disclosure shows a demethylation state similar to that of nTreg.

It is shown that the inducible regulatory T cell obtained from the above examples is, unlike endogenous nTreg, a regulatory T cell newly induced in vitro, but has characteristics that are not present in existing inducible regulatory T cells having high induction efficiency, high CTLA4 expression, and demethylation of the FOXP3 CNS2 region.

### (Example 2: In vitro suppressive activity of regulatory T cell)

A regulatory T cell was obtained by the same experimental system as in Example 1. The responder T cells (5 × 10⁴) labeled with Cell Trace Violet and the prepared regulatory T cells (5 × 10⁴) were mixed and cultured for 3 days in the presence of a Treg Suppression Inspector (Miltenyi Biotec) (5 µL/well), and the Cell Trace Violet intensity was analyzed by a flow cytometry method. When the responder T cell develops an immune reaction, the Cell Trace Violet intensity shows a plurality of weak peaks due to cell proliferation, but when this immune reaction is suppressed, the Cell Trace Violet intensity is maintained as a single strong peak. It was confirmed that the regulatory T cell obtained by the preparation method of the present disclosure strongly suppresses the immune reaction of the responder T cell. Therefore, it was confirmed that the obtained regulatory T cell has a higher suppression function than the existing regulatory T cell population (FIG. 3).

### (Example 3: Comprehensive gene expression analysis of regulatory T cell)

The gene expression pattern of the regulatory T cell prepared by the method of Example 1 was confirmed by an RNA sequencing method (FIG. 4). The RNA sequencing was performed using a known method (Kitagawa et al. (2017) Nat. Immunol. 18, 173-183). Genes of major suppressive molecules (such as CTLA4) of FOXP3, IL-2RA(CD25), and Treg were confirmed to be highly expressed in the inducible regulatory T cell obtained by the preparation method of the present disclosure and the nTreg cell compared with the existing inducible regulatory T cell and the activated Tconv cell. It was confirmed that expression of IKZF2 as a marker of the nTreg cell was low in the inducible regulatory T cell. It can be confirmed that the inducible regulatory T cell obtained by the preparation method of the present disclosure has higher expression of some suppressive molecules such as ENTPD1, NT5E, and AREG as compared with other regulatory T cell populations. It can be confirmed that expression of adhesion molecules such as ITGAE and chemokine receptors such as CCR4 is high (FIG. 4).

As an example, when the expression of a CD103 molecule encoded by ITGAE is analyzed by a flow cytometry method, it can be confirmed that the inducible regulatory T cell obtained by the preparation method of the present application has higher CD 103 expression as compared with other regulatory T cell populations (FIG. 5). As a comprehensive consideration, it can be expected that the inducible regulatory T cell obtained by the preparation method of the present application exhibits a high suppressive ability as compared with the existing regulatory T cell.

### (Example 4: Preparation of regulatory T cell from CD8-positive human peripheral T cell)

The CD8-positive T cell was stimulated for 3 days in a basal medium containing an anti-CD3 antibody (the antibody having a concentration of 10 µg/ml is added in an amount of 100 µL per well, and is allowed to stand at room temperature for 60 minutes to be immobilized on the container), hIL-2 (100 U/ml), hTGFβ1 (10 ng/ml), retinoic acid (1 µM), and Senexin A (5 µM). Thereafter, the cell was subjected to dormant culturing in a basal medium containing hIL-2 (100 U/ml) for 2 days. After 2 days, the medium was replaced in the same manner, and dormant culturing was further performed for 2 days. After 2 days, the cell was stimulated again for 2 days in a basal medium containing an anti-CD3 antibody (the antibody having a concentration of 10 µg/ml is added in an amount of 100 µL per well, and is allowed to stand at room temperature for 60 minutes to be immobilized on the container), hIL-2 (100 U/ml), hTGFβ1 (10 ng/ml), retinoic acid (1 µM), Senexin A (5 µM), and ascorbic acid (10 µg/ml). Thereafter, the cell was subjected to dormant culturing in a basal medium containing hIL-2 (100 U/ml) for 2 days. After 2 days, the medium was replaced in the same manner, and dormant culturing was further performed for 2 days. The expression of FoxP3, CTLA4, and Helios in the regulatory T cell obtained after 2 days was analyzed by a flow cytometry method (FIG. 6).

The inducible regulatory T cell of the present disclosure prepared from the CD8-positive T cell was Helios-negative, and could be confirmed to be a population containing many cells expressing FoxP3 and CTLA4.

### (Example 5: Preparation of inducible regulatory T cell from human peripheral T cell)

An inducible regulatory T cell or a cell population thereof are obtained in the same manner as in Example 1 using a human peripheral T cell. A pharmaceutical comprising this inducible regulatory T cell or the cell population thereof and a carrier is produced. This pharmaceutical comprises about 2 × 10⁵ cells as inducible regulatory T cells or a cell population thereof, and the efficacy can be confirmed from the degree of inflammation in tissues collected 1 month after administration to a patient with an inflammatory disease.

### (Example 6: Formulation Example)

An inducible regulatory T cell or a cell population thereof are obtained in the same manner as in Example 1. A pharmaceutical comprising this inducible regulatory T cell or the cell population thereof and a carrier is produced.

### (Example 7: Expression intensity analysis)

For the cell population of the inducible regulatory T cell prepared in Example 1, the expression intensity of CD25 and FOXP3 was analyzed by a flow cytometry method using a BD FACSLyric flow cytometer. The results are shown in FIG. 7.

In all the tested cells, it could be confirmed that the cell population of FOXP3^{Hi} inducible regulatory T cells was present at a high proportion.

### (Example 8: Antibody panel analysis of inducible regulatory T cell)

In order to comprehensively analyze the cell surface marker in the inducible regulatory T cell prepared in Example 1, antibody panel analysis was performed. The inducible regulatory T cell prepared in Example 1 was stained using LEGENDScreen Human PE Kit of BioLegend, and the antibody reacting with the inducible regulatory T cell of the present disclosure was analyzed by a flow cytometry method. Some of the results (clear and meaningful expression could be confirmed) are shown in Tables 1 to 4.

When the inducible regulatory T cell was stained with an antibody panel containing slightly less than 400 kinds, positive/negative features of the cell surface markers in the inducible regulatory T cell of the present disclosure could be confirmed by at least 185 kinds of surface marker molecules.

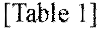

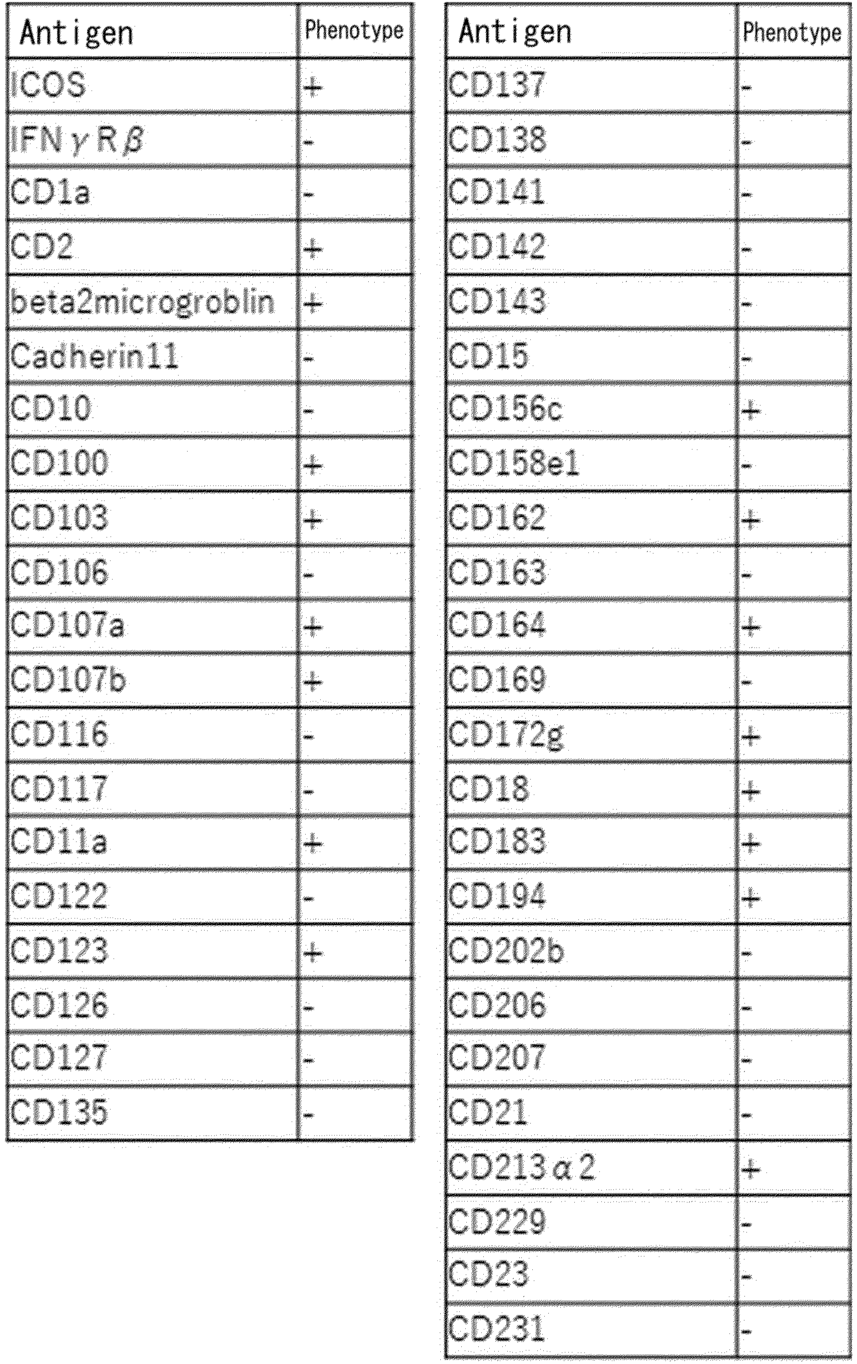

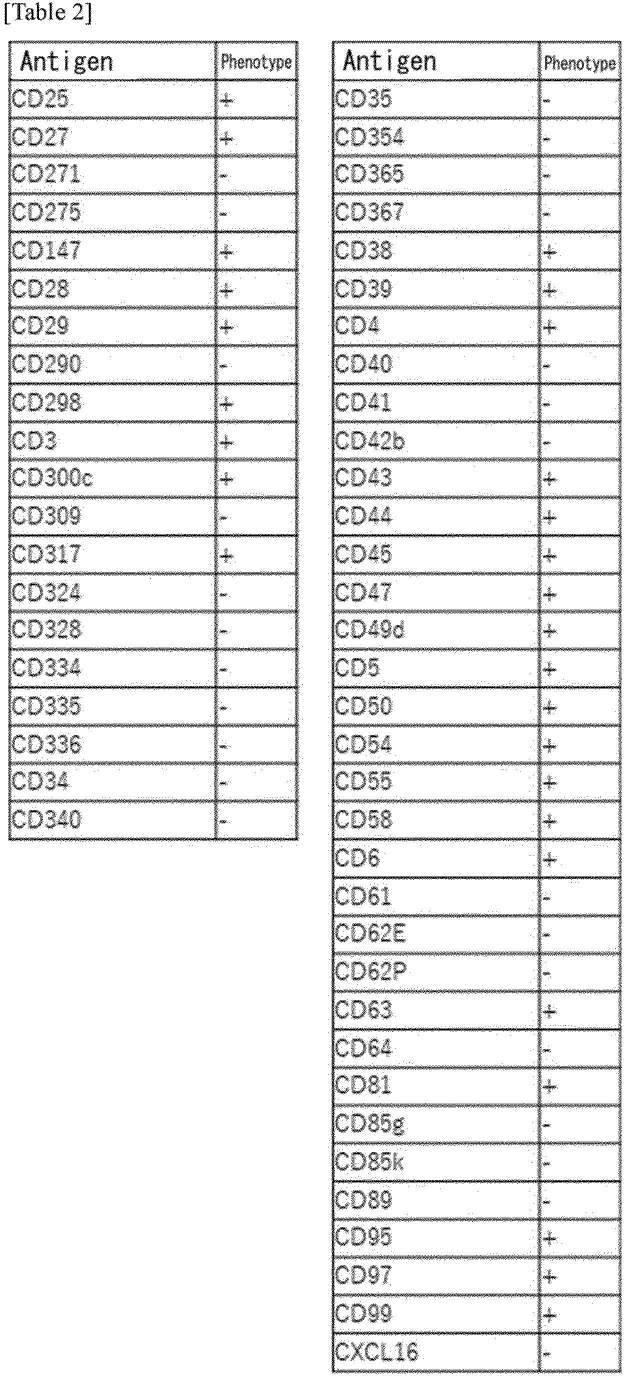

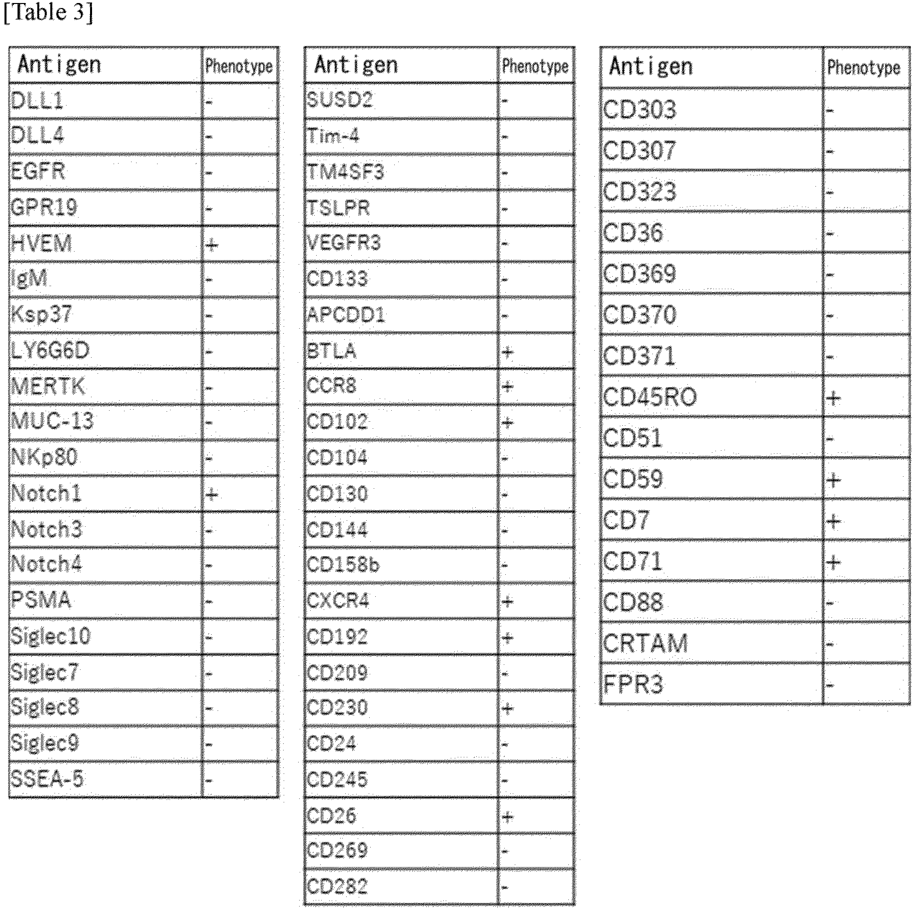

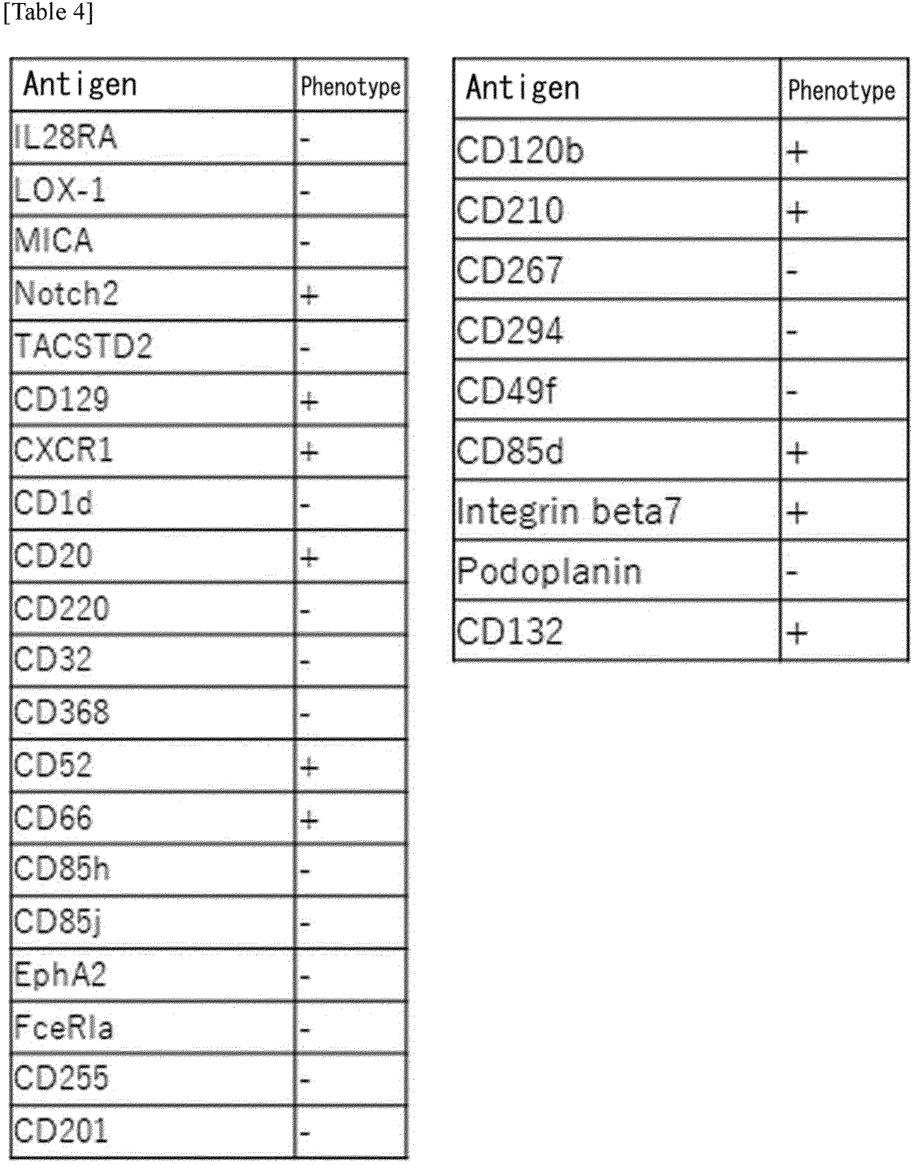

### (Example 9: cell surface marker analysis in inducible regulatory T cell)

Characteristic results of antibody screening performed in Example 8 are shown in FIG. 8.

In the inducible regulatory T cell of the present disclosure, specific cell surface markers such as CD172g and CD26 were found to be positive.

### (Note)

As described above, the present disclosure has been exemplified by the use of its preferred embodiments; however, it is understood that the scope of the present disclosure should be interpreted solely based on the claims. It is also understood that any patent, any patent application, and any references cited in the present specification should be incorporated in the present specification by reference in the same manner as the contents are specifically described in the present specification. The present application claims priority to Japanese Patent Application No. 2021-190127 filed on November 24, 2021 with the Japan Patent Office, the entire contents of which are incorporated herein by reference in the same manner as if the contents are specifically described herein.

### Industrial Applicability

The inducible regulatory T cell and/or the cell population of the present disclosure can be used for treatment or prevention of various immune diseases and inflammatory diseases such as autoimmune diseases. It is possible to stably induce a regulatory T cell having high functionality from a peripheral T cell by the method for preparing the inducible regulatory T cell and/or the cell population of the present disclosure, and application to the medical fields can be expected.

### Sequence Listing Free Text

SEQ ID NO: 1: Forward primer used in Examples
SEQ ID NO: 2: Reverse primer used in Examples

## Claims

1. An inducible regulatory human T cell having at least one feature selected from the group consisting of FoxP3 positivity, CTLA4 positivity, NT5E positivity, ITGAE (CD103) positivity, AREG positivity, CD172g positivity, and CD26 positivity.

2. An inducible regulatory human T cell which is NT5E-positive.

3. An inducible regulatory human T cell which is ITGAE (CD103)-positive.

4. An inducible regulatory human T cell which is AREG-positive.

5. An inducible regulatory human T cell which is CD172g-positive.

6. An inducible regulatory human T cell which is CD26-positive.

7. An inducible regulatory human T cell which is CTLA4-positive.

8. The inducible regulatory human T cell according to any one of claims 1 to 7, which is at least CTLA4-positive and FoxP3-positive.

9. The inducible regulatory human T cell according to any one of claims 1 to 8, which is at least CD172g-positive and/or CD26-positive.

10. The inducible regulatory human T cell according to any one of claims 1 to 9, wherein the CNS2 site of the FOXP3 gene is demethylated.

11. The inducible regulatory human T cell according to any one of claims 1 to 10, which is CD4-positive or CD8-positive.

12. The inducible regulatory human T cell according to any one of claims 1 to 11, which is obtained from or derived from a human peripheral blood T cell or a human tissue-derived T cell.

13. An inducible regulatory human T cell obtained by a method including the steps of:
(a) stimulating a CD4-positive T cell or a CD8-positive T cell in human peripheral blood in a first basal medium for about 1 to about 5 days;
(b) dormant culturing the cell obtained in step (a) in a medium containing IL-2 for at least about 1 to about 3 days;
(c) stimulating the cell obtained in step (b) in a second basal medium for about 1 to about 5 days; and
(d) dormant culturing the cell obtained in step (c) in a medium containing IL-2 for at least about 1 to about 3 days.

14. A cell population comprising the inducible regulatory human T cell according to any one of claims 1 to 13, wherein
the cell population has a proportion of at least one feature selected from the group consisting of FoxP3 positivity, CTLA4 positivity, NT5E positivity, ITGAE (CD103) positivity, AREG positivity, CD172g positivity, and CD26 positivity of about 50% or more.

15. A cell population comprising an inducible regulatory human T cell, wherein
the cell population has a proportion of NT5E positivity of about 50% or more.

16. A cell population comprising an inducible regulatory human T cell, wherein
the cell population has a proportion of ITGAE (CD103) positivity of about 50% or more.

17. A cell population comprising an inducible regulatory human T cell, wherein
the cell population has a proportion of AREG positivity of about 50% or more.

18. A cell population comprising an inducible regulatory human T cell, wherein
the cell population has a proportion of CD172g positivity of about 50% or more.

19. A cell population comprising an inducible regulatory human T cell, wherein
the cell population has a proportion of CD26 positivity of about 50% or more.

20. A cell population comprising an inducible regulatory human T cell, wherein
the cell population has a proportion of CTLA4 positivity of about 50% or more.

21. The cell population according to any one of claims 14 to 20, wherein the proportions of at least CTLA4 positivity and FoxP3 positivity each are about 50% or more.

22. The cell population according to any one of claims 14 to 21, wherein the proportions of at least CD172g positivity and/or CD26 positivity each are about 50% or more.

23. The cell population according to any one of any one of claims 14 to 22, wherein the proportion of at least one feature in the cell population is about 60% or more.

24. The cell population according to any one of claims 14 to 23, wherein the proportion of at least one feature in the cell population is about 80% or more.

25. The cell population according to any one of claims 14 to 24, wherein the proportion of FoxP3 strong positivity is about 50% or more.

26. The cell population according to any one of claims 14 to 25, wherein about 90% or more of the cell population is T cells.

27. A pharmaceutical composition comprising the inducible regulatory human T cell according to any one of claims 1 to 13 or the cell population according to any one of claims 14 to 26.

28. A regenerative medical material or product comprising the inducible regulatory human T cell according to any one of claims 1 to 13 or the cell population according to any one of claims 14 to 26.

29. A method for preparing an inducible regulatory human T cell, the method comprising the steps of:
(a) stimulating a CD4-positive T cell or a CD8-positive T cell in human peripheral blood in a first basal medium for about 1 to about 5 days;
(b) dormant culturing the cell obtained in step (a) in a medium containing IL-2 for at least about 1 to about 3 days;
(c) stimulating the cell obtained in step (b) in a second basal medium for about 1 to about 5 days; and
(d) dormant culturing the cell obtained in step (c) in a medium containing IL-2 for at least about 1 to about 3 days.

30. The method according to claim 29, wherein the first basal medium comprises at least one factor selected from the group consisting of an anti-CD3 antibody, TGF-β1, IL-2, retinoic acid, a CDK8 inhibitor, a CDK19 inhibitor, a CDK8/19 inhibitor, and ascorbic acid.

31. The method according to claim 29 or 30, wherein the first basal medium comprises an anti-CD3 antibody, TGF-β1, IL-2, retinoic acid, a CDK8 inhibitor, a CDK19 inhibitor, a CDK8/19 inhibitor, and ascorbic acid.

32. The method according to any one of claims 29 to 31, wherein the second basal medium comprises at least one factor selected from the group consisting of an anti-CD3 antibody, TGF-β1, IL-2, retinoic acid, a CDK8 inhibitor, a CDK19 inhibitor, and a CDK8/19 inhibitor.

33. The method according to any one of claims 29 to 32, wherein the second basal medium comprises an anti-CD3 antibody, TGF-β1, IL-2, retinoic acid, a CDK8 inhibitor, a CDK19 inhibitor, and a CDK8/19 inhibitor.

34. The method according to any one of claims 29 to 33, wherein step (a) is to stimulate the CD4-positive T cell or the CD8-positive T cell in the first basal medium for about 3 days.

35. The method according to any one of claims 29 to 34, wherein step (b) is to dormant culture the cell obtained in step (a) in the medium containing IL-2 for at least about 2 days.

36. The method according to any one of claims 29 to 35, wherein step (c) is to stimulate the cell obtained in step (b) in the second basal medium for about 3 days.

37. The method according to any one of claims 29 to 36, wherein step (d) is to dormant culture the cell obtained in step (c) in the medium containing IL-2 for at least about 2 days.

38. An inducible regulatory human T cell generated by the method according to any one of claims 29 to 37 or a cell population comprising the inducible regulatory human T cell.
